# EUROPEAN PATENT APPLICATION

(11) **EP 4 427 752 A1**
(43) Date of publication of application: **11.09.2024**
(21) Application number: 23382223.8
(22) Date of filing: 09.03.2023
(51) Int. Cl.: A61K 31/519, A61P 19/02, A61P 21/00, A61P 29/00

(54) **DIPYRIDAMOLE AS A NOVEL THERAPY FOR MUSCULAR MYOGENESIS DISORDERS AND INFLAMMATORY ARTHRITIS**

(71) Applicant: Fundacion Instituto De Investigacion Sanitaria Fundacion Jimenez Diaz, 28040 Madrid (ES)
(72) Inventor: MEDIERO MUÑOZ, Aránzazu, 28040 Madrid (ES); MARCO BONILLA, Miguel, 28040 Madrid (ES); FRESNADILLO CASTAÑO, María, 28040 Madrid (ES); LARGO CARAZO, Raquel, 28040 Madrid (ES); HERRERO-BEAUMONT CUENCA, Gabriel, 28040 Madrid (ES)
(74) Representative: Hoffmann Eitle

(57) **Abstract**

The present invention relates to the use of dipyridamole in a method of treatment, amelioration or prevention of muscular myogenesis disorders, specifically sarcopenia. The invention also relates to the use of dipyridamole in a method of treatment, amelioration or prevention of inflammatory arthritis.

## Description

### TECHNICAL FIELD

The present invention relates to the use of dipyridamole in a method of treatment, amelioration or prevention of muscular myogenesis disorders, specifically sarcopenia. The invention also relates to the use of dipyridamole in a method of treatment, amelioration or prevention of inflammatory arthritis.

### BACKGROUND OF THE INVENTION

Sarcopenia is a condition characterized by a progressive and generalized loss of skeletal muscle mass and function with an increased risk of adverse outcomes such as disability, metabolic dysfunction, poor quality of life, and death (1). After the age of 30, an individual loses between 3 and 8% of muscular mass every decade, increasing this rate after the age of 60 (2). This decrease in muscle mass produces a decline in strength and muscular function, with qualitative changes in muscular tissue due to a reduction in motor units affecting both nervous and muscular fibers, especially fiber type II, altering therefore the contractile activity (3,4). According to the European Working Group on Sarcopenia in Older People (EWGSOP), the presence of low skeletal muscle mass (e.g., DXA, Anthropometry) and either low muscle strength (e.g., handgrip, isokinetic) or low muscle performance (e.g., walking speed, muscle power), which is often related to the most advanced stages of sarcopenia, are the criteria for the diagnosis of sarcopenia (5,6).

Even though sarcopenia is mainly associated with the aging process seen in the elderly (primary sarcopenia), there are several other populations at risk due to lifestyle decisions or pathological states. Secondary sarcopenia can be considered when one or more of the following causes are evident: sedentary, immobilization, malnutrition, diabetes, obesity, and other acute or chronic inflammatory diseases (e.g. Rheumatoid sarcopenia) that could promote the loss of muscle mass (7,8).

Muscle loss in both primary and secondary sarcopenia appears to be driven by different mechanism, as sarcopenia in elderly is primarily produced by anabolic resistance produced by Myostatin (9), meanwhile secondary sarcopenia appears to be driven by catabolic processes. Under normal circumstances, IL-6, TNF-α and C-reactive protein (CRP) maintain the balance between synthetic metabolism and catabolism of skeletal muscle, but higher levels of inflammatory markers are associated with physical decline, resulting in increased catabolism, and they can also inhibit protein synthesis of skeletal muscle cells, damage muscle integrity and function, thereby resulting in sarcopenia (10,11). Anti-inflammatory cytokines (IL-4, IL-10 and IL-15) can antagonize the expression and activity of these pro-inflammatory cytokines to reduce muscle atrophy and retard sarcopenia (12). In addition, IL-4 can improve glucose metabolism in muscle cells and act as a myoblast recruitment factor during mammalian muscle growth, to promotes myogenesis and muscle regeneration (13,14).

In this regard, sarcopenia can be observed in chronic debilitating diseases, as well as in typical inflammatory conditions, but in these cases, the pattern of sarcopenia depends on the severity of the injury (15).

Rheumatoid arthritis (RA) is a chronic, inflammatory, and autoimmune disease of unknown etiology that causes destruction of joint cartilage and bone and reduced life expectancy (16). Both genetic and environmental components contribute to the etiology of RA and together lead to an early immune alteration in both innate and adaptative compartments (17). Early cartilage and bone erosions are associated with accumulation of inflammatory cells in the synovial membrane, including macrophages, T and B lymphocytes, dendritic cells, and polymorphonuclear leukocytes, which mediate the destructive changes in the synovium (18). Despite marked improvements in the treatment of RA and other forms of inflammatory arthritis, the pathogenesis of inflammatory arthritis remains incompletely elucidated and, for many patients, novel approaches for antirheumatic therapies are needed.

In addition to joint damage, RA confers a higher risk of several comorbidities, such as cardiovascular disease and infections (19). Moreover, changes in body composition including reduced fat-free mass, with muscle mass as the major component, and stable or increased fat mass, results in limited changes in body mass index (20,21). This condition is known as rheumatoid cachexia (RC). Loss of body cell mass is independent of duration of disease, therapy, or energy intake (21,22). A recent meta-analysis showed that RC prevalence varies between 1% and 54%, there is no consensus on the clinical criteria for its diagnosis and it is correlated with a high risk of physical disability, morbidity, and mortality (23).

Muscle homeostasis is maintained through an accurate equilibrium between anabolic and catabolic processes. A shift in this equilibrium to inflammatory-mediated catabolism appears to be the key factor in RC (24). The consensus between pro/anti-anabolic and anti-catabolic factors determines the yield of muscle turnover (25). Unfortunately, current therapies fail reverting RC despite achieving RA remission. The hypercatabolic status of RA patients leads to a 'permanent footprint' that persists even after controlling disease activity (26).

During inflammation, there is a shift in skeletal muscle homeostasis toward muscle loss. It is thought that an overacting immune system can divert energy expenditure and lead to a shortage of stored reserves affecting general metabolism (15).

The regulation of cellular energy homeostasis by phosphorylation of ADP to ATP is critical. These molecules not only serve as an energy reservoir; they can also act as extracellular messengers. The purinergic system is a signaling system, where the purine nucleotides, ATP, ADP and the nucleoside, adenosine, act as extracellular messengers (27). Adenosine is generated both intracellularly and extracellularly from the hydrolysis of adenine nucleotides and acts locally to exert its extracellular physiologic and pharmacologic effects via activation of specific cell surface G protein coupled receptors (A1, A2A, A2B and A3), proteins with unique pharmacological profile, tissue distribution and effectors coupling (28,29). Purine and pyrimidine nucleotides can activate two classes of receptors: ligand-gated ion channels (P2X receptor, ionotropic receptors) or G protein coupled receptors (P2Y receptors, metabotropic receptors). (30,31). The following purinergic receptors are expressed in human skeletal muscle fibers: P2X1, P2Y11, P2Y4 and A2A and A2B adenosine receptor (32-35). During high intensity muscle contractions, ATP is rapidly degraded, leading to enhanced intracellular production of AMP and adenosine (36). Adenosine receptor activation is important for insulin to be able to exert its stimulatory action on glucose transport in muscle subjected to contractile activity with A2A receptor leading this function (36). A recent work has demonstrated that A2B receptor is the most highly expressed adenosine receptor in human and murine skeletal muscle cells (as well as brown adipose tissue (BAT)) and that A2B mediates the largest part of the adenosineinduced increase in cAMP in this tissue and might be targeted to counteract age-related sarcopenia as well as obesity (37). One important mechanism observed during the myotube formation is that the level of intracellular cAMP, the activity of PKA and the phosphorylation of CREB were downregulated (38).

In addition, the paracrine signaling that regulates proliferation, migration, differentiation etc. in muscle cells is regulated by cytoskeletal rearrangements and cell-cell communication, mediated in part by Pannexins, a family of transmembrane channel proteins that mediate paracrine signaling by ATP release (39). Among the three members of the family, Panx-1 and Panx-3, but not Panx-2, are present in both murine and human skeletal muscle and are co-expressed.

It has been previously demonstrated by the inventors that treatment with agents that increase adenosine levels due to blockade of its cellular uptake such as dipyridamole has beneficial effects in bone homeostasis depending on intact adenosine A2A receptor signaling (40-42). Recent data of the inventors have furthermore demonstrated that dipyridamole reverts the bone deleterious effect of tenofovir, an AMP analog and one of the most commonly used antivirals in HIV, which blocks ATP release by Pannexin-1 (43), both in vitro and in vivo (44). Previous studies have shown that mice treated with tenofovir at 75mg/Kg/day lost nearly 10% of body weight which was recovered when mice were treated with dipyridamole at 25mg/Kg/day (44). Dipyridamole also reverses the tenofovir induced increase in PCR serum levels. This was attributed to a cachexia state due to the decrease of extracellular ATP concentrations that produce the reduction in % fat body composition as well leading to sarcopenia. Dipyridamole corrected the body weight possibly due to the increase in extracellular adenosine levels (44).

Understanding the mechanisms activated by the purinergic system involved in sarcopenia and rheumatoid arthritis is clinically relevant and provides the opportunity to develop novel treatment strategies.

The use of adenosine receptor agonists and antagonists or dipyridamole in the regulation of osteoblast differentiation, proliferation and function is known. Dipyridamole is described in the prior art to increase adenosine to stimulate adenosine A2B receptors to stimulate osteoblast production of bone matrix and inhibit IL-6 production or increase production of osteoprotegerin (45-49). PCT/US2013/027097 teaches using adenosine A1 or A2A receptor agonists or antagonists or dipyridamole for the treatment of bone defects following trauma or to promote spinal fusion.

Whilst dipyridamole is a commercially available pharmacological compound, it to date has not been described as a treatment option for diseases having their cause in muscular myogenesis disorders, such as for example sarcopenia, or as a potential treatment for inflammatory arthritis, such as for example rheumatoid arthritis.

With an ever-ageing population there is an increasing need for novel treatment options for muscular myogenesis disorders as well as inflammatory arthritis.

### BRIEF DESCRIPTION OF THE FIGURES

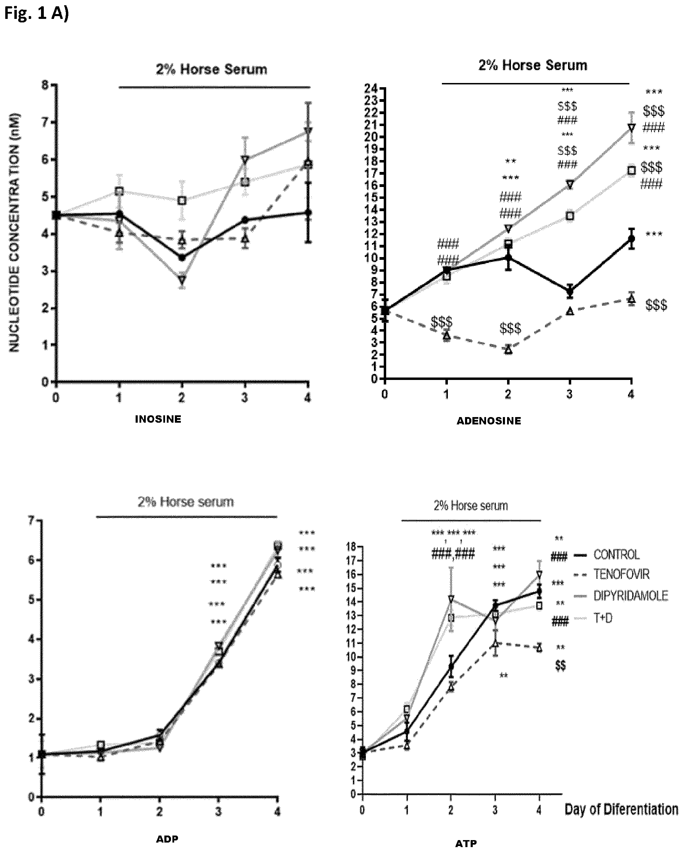

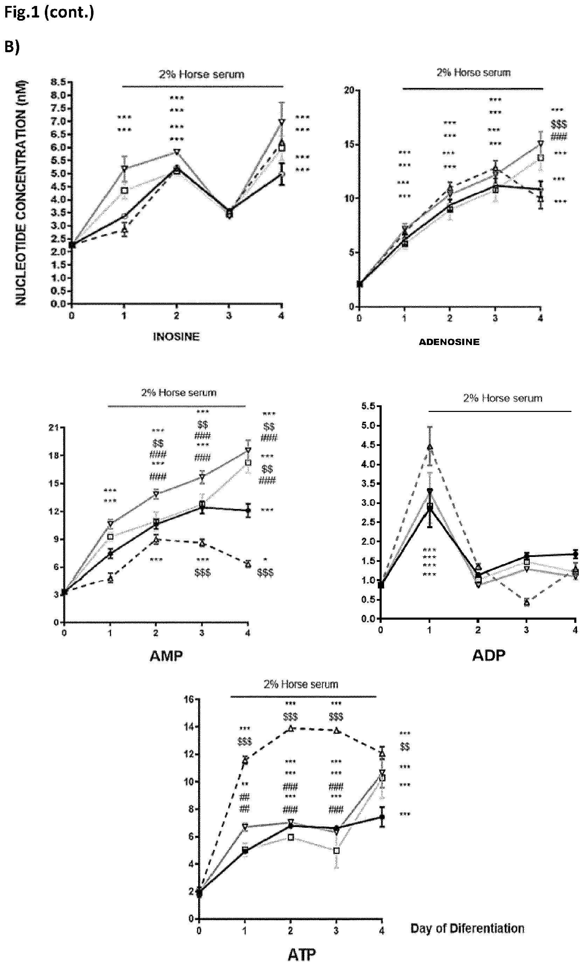

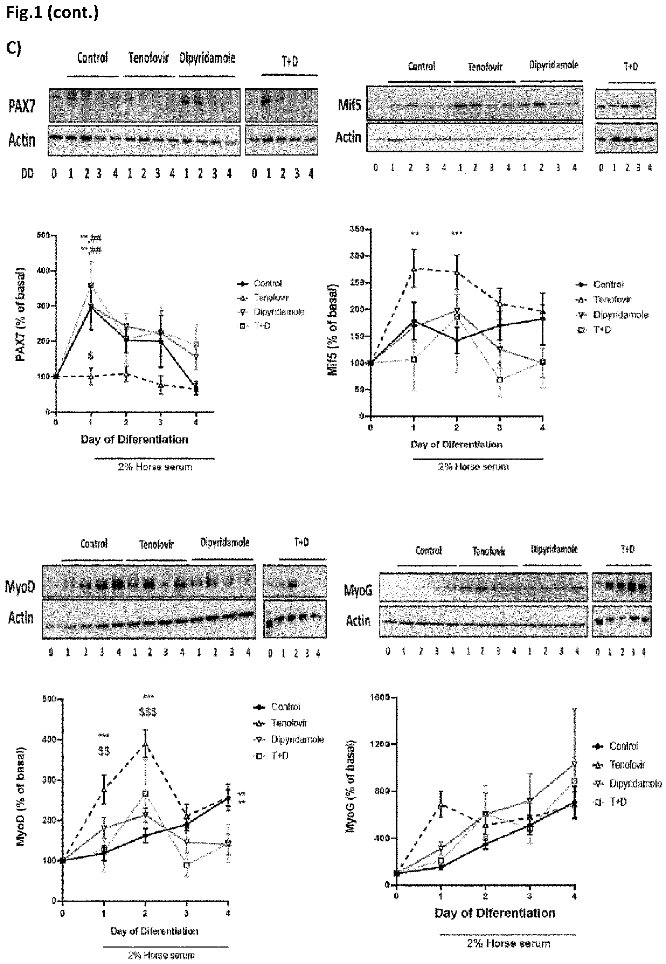

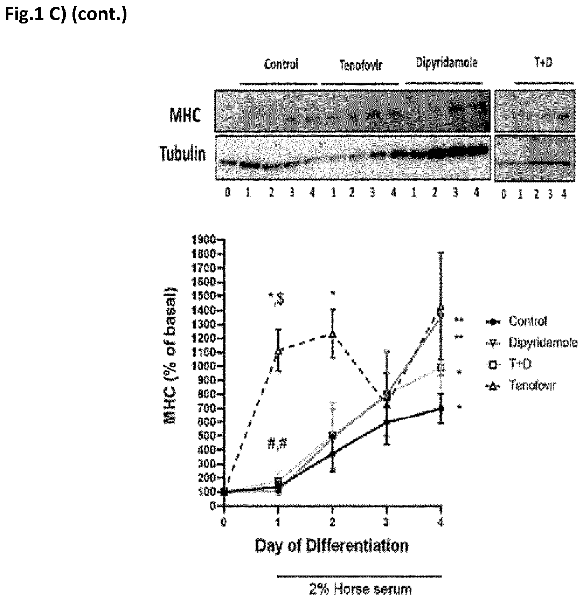

Fig.1: Analysis by high performance liquid chromatography (HPLC) during 4 days of differentiation in the presence of tenofovir and dipyridamole (A) Extracellular concentration of inosine, adenosine, ADP and ATP. (B) Intracellular concentration of inosine, adenosine, AMP, ADP and ATP by HPLC. (C) Western blot analysis of myoblast/myotube markers. One representative of n=5 per group and day is displayed. Data is mean±SEM. ^{∗}Treatment vs basal. T+D: tenofovir + dipyridamole; DD: days of differentiation.

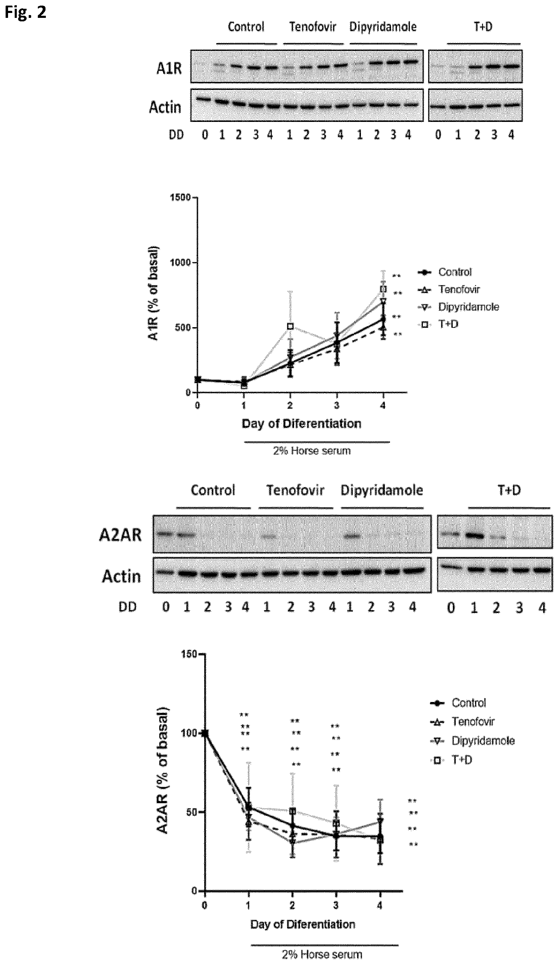

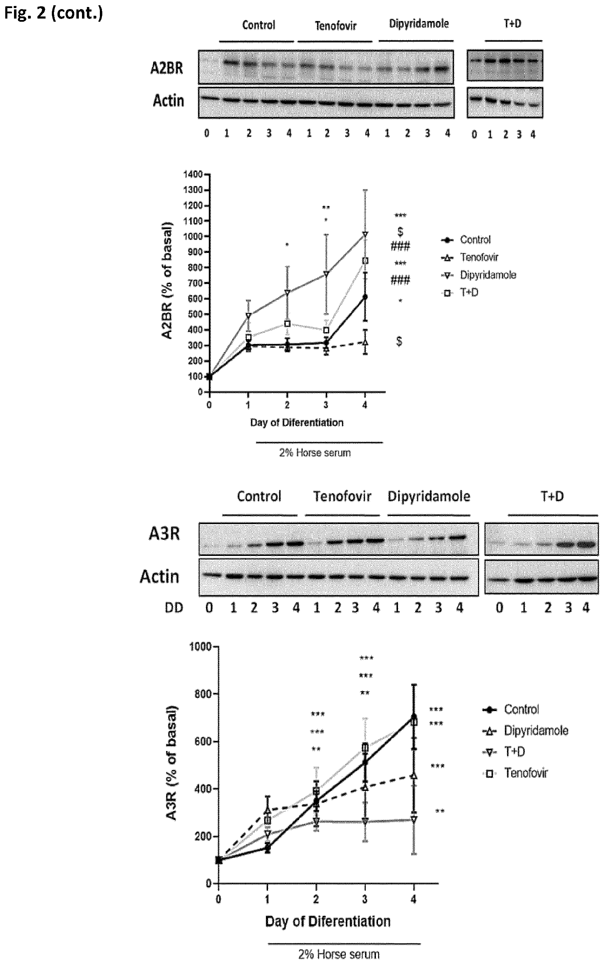

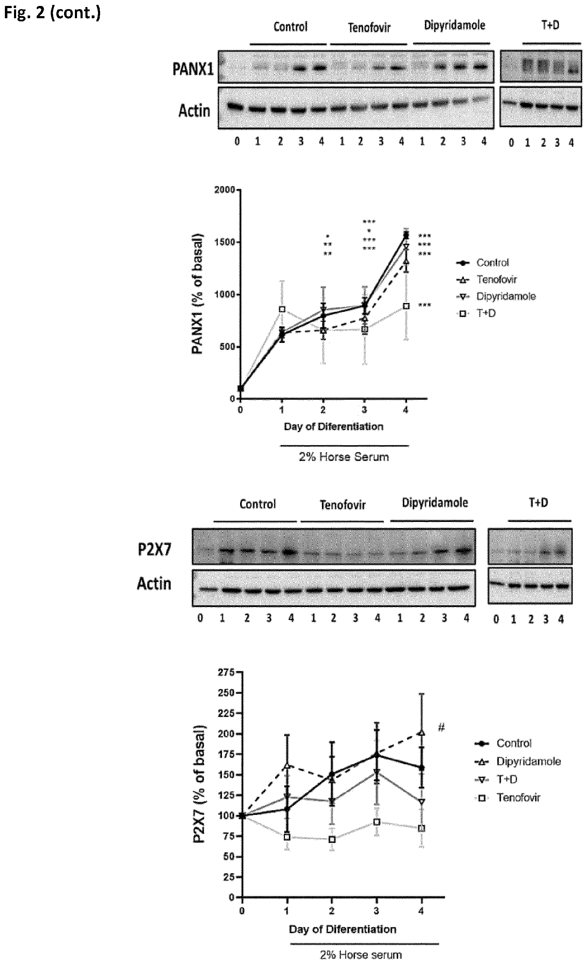

Fig.2: Western blot protein expression during the 4 days of differentiation in the presence of tenofovir and dipyridamole. Protein expression of A1, A2A, A2B and A3 adenosine receptors, P2X7 receptor and Panx-1. Data are representative of n=5 each group per day (mean ± SEM). ^{∗} Treatment vs basal, $ Tenofovir and dipyridamole vs. control and # dipyridamole and T+D vs tenofovir. T+D: tenofovir + dipyridamole; DD: days of differentiation.

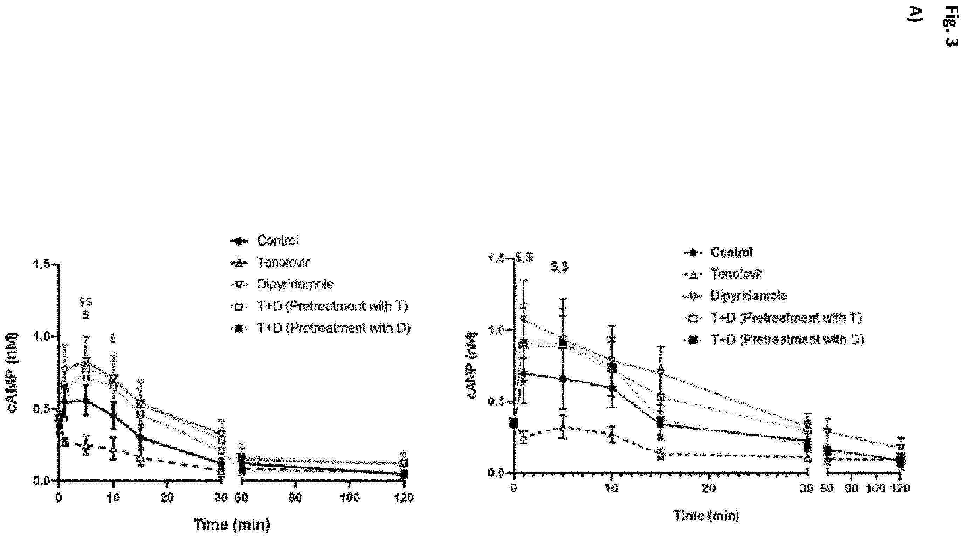

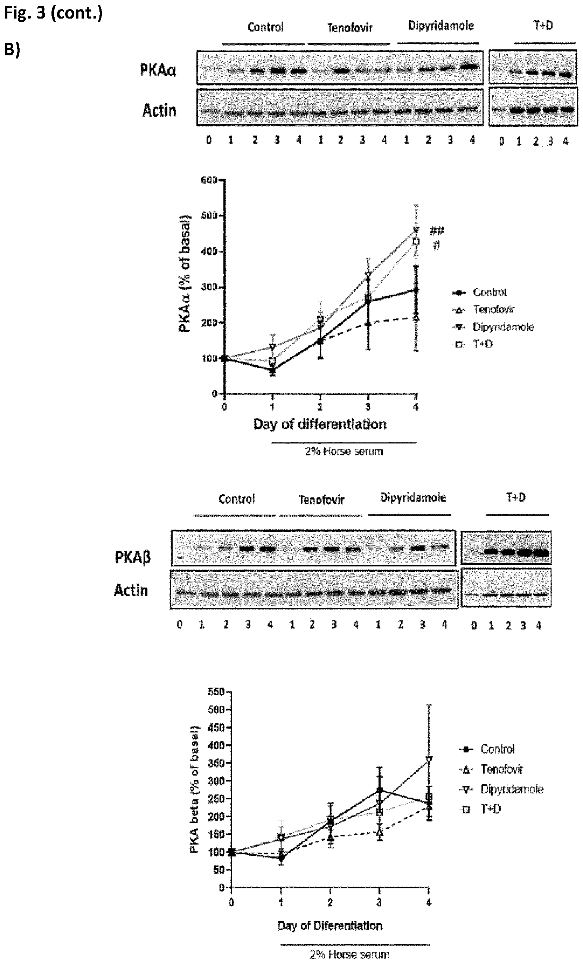

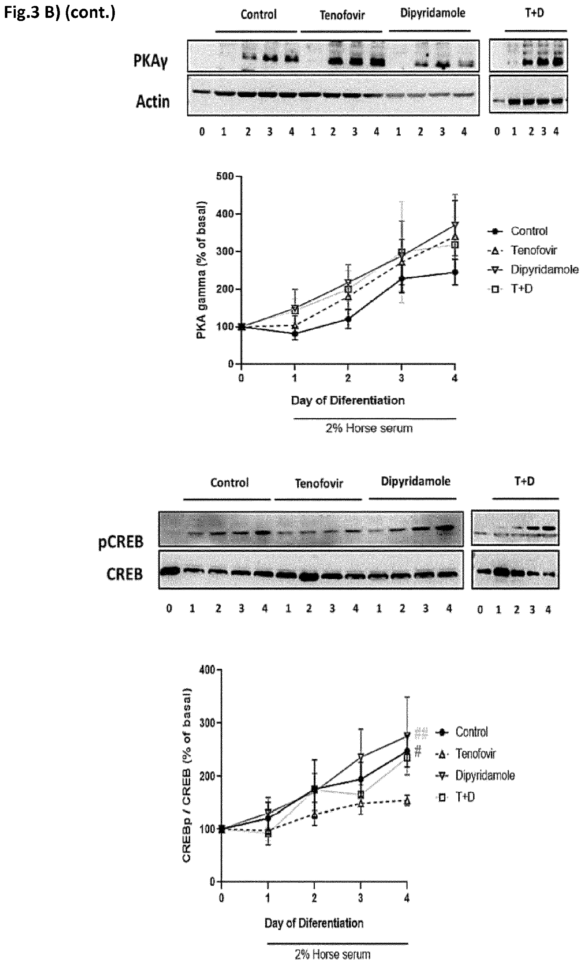

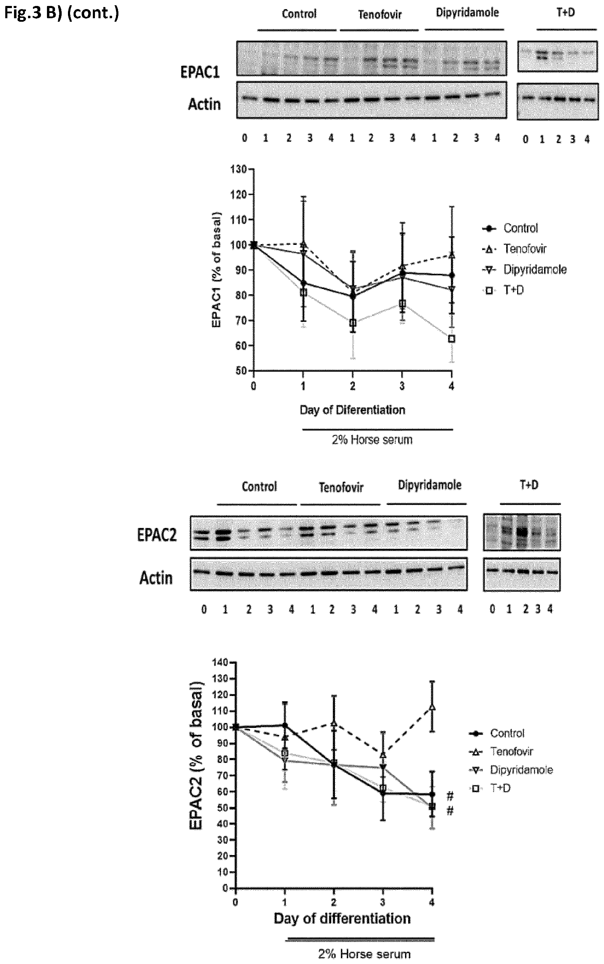

Fig.3: cAMP/PKA pathway and AMPK activation in the presence of dipyridamole. (A) cAMP levels in myoblast (left) and in myotube (right). (B) Study of protein expression of the PKAα, PKAβ and PKAγ, pCREB and EPAC 1 and 2. Data are representative of n=5 each group per day (mean ± SEM). * Treatment group vs. control group, $ treatment group vs. tenofovir group. T+D: tenofovir + dipyridamole; DD: days of differentiation.

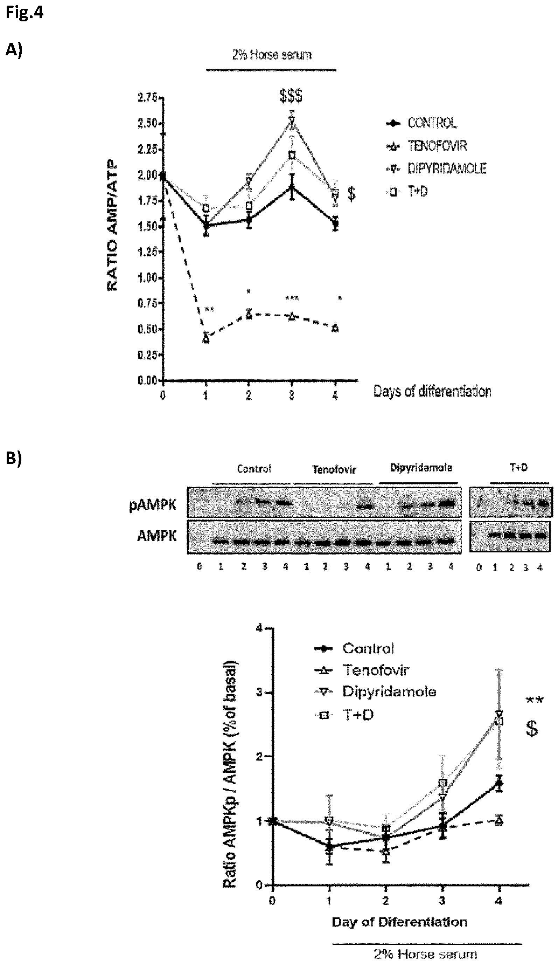

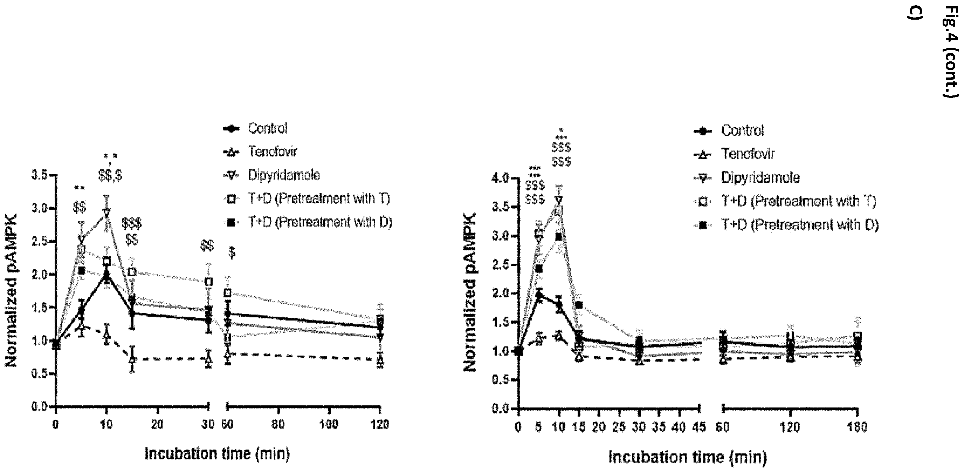

Fig.4: AMPK activation mediated by dipyridamole. (A) Ratio of intracellular AMP/ATP concentration. (B) Expression of phosphorylated AMPK (AMPKp) and (C) AMPKp levels by ELISA in myoblast (left) and in myotube (right) Data are represented as mean ± SEM for n=5-7 (western blot and HPLC) or n=9-12 (AMPK ELISA) per group and and day. ^{∗}Treatment group vs. control group, $ treatment group vs. tenofovir group. T+D: tenofovir + dipyridamole; DD: days of differentiation.

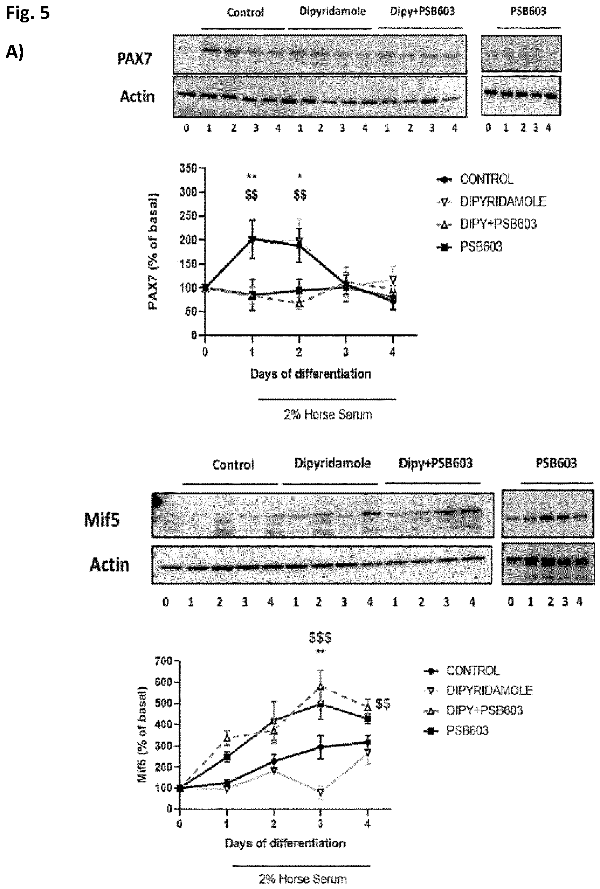

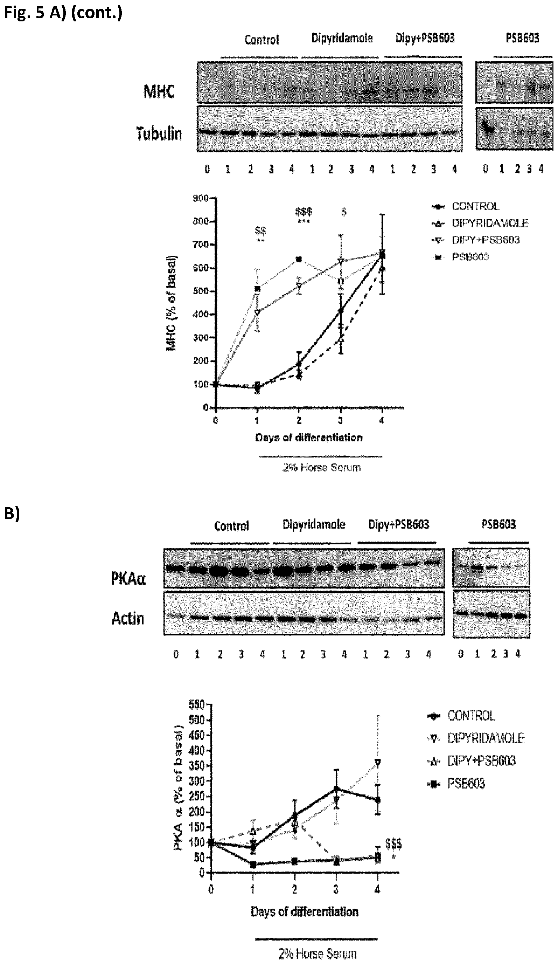

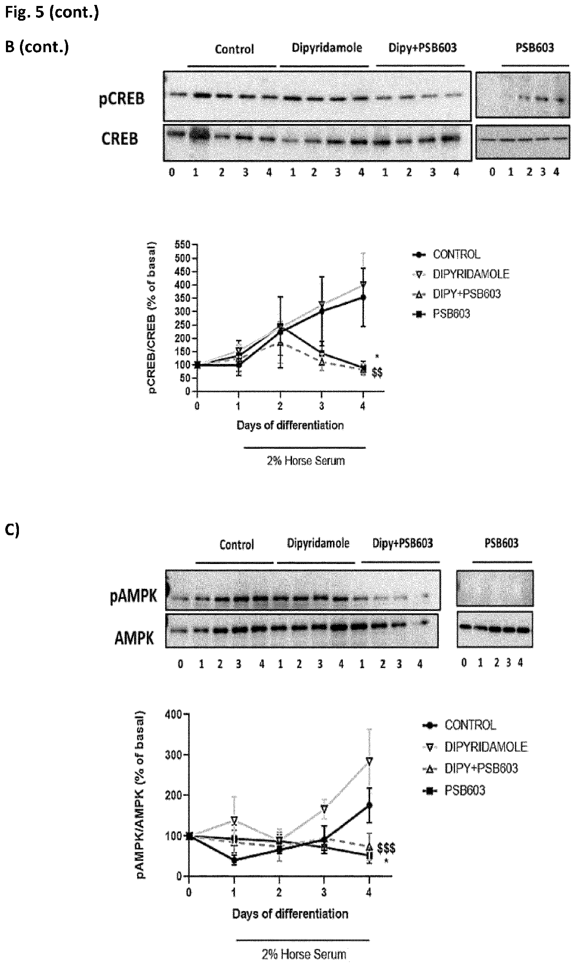

Fig.5: Dipyridamole effect in myogenesis is dependent on the activation of adenosine A2B receptor. (A) Analysis of protein expression for 4 days with 2% HS + dipyridamole ± PSB-603 of PAX7, Mif5 and MHC muscle proliferation/differentiation markers, (B) protein expression of PKAα and pCREB for 4 days with 2% HS + dipyridamole ± PSB-603 and (C) phosphorylated AMPK for 4 days with 2% HS + dipyridamole ± PSB-603. Analysis is represented as mean±SEM n=5 per group and day. * Treatment vs control, $dipyridamole vs dipy+PSB-603; DD: days of differentiation.

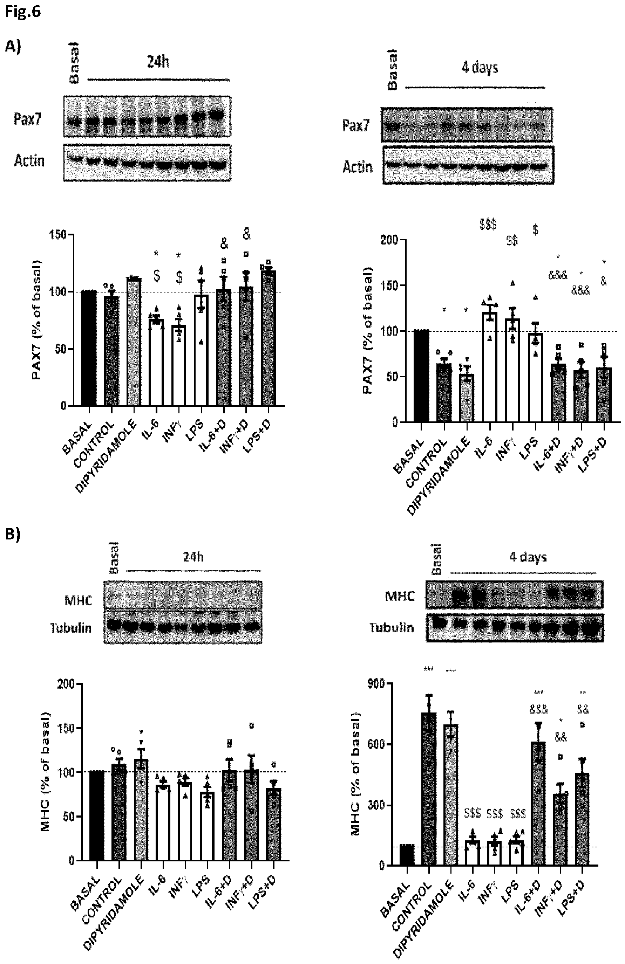

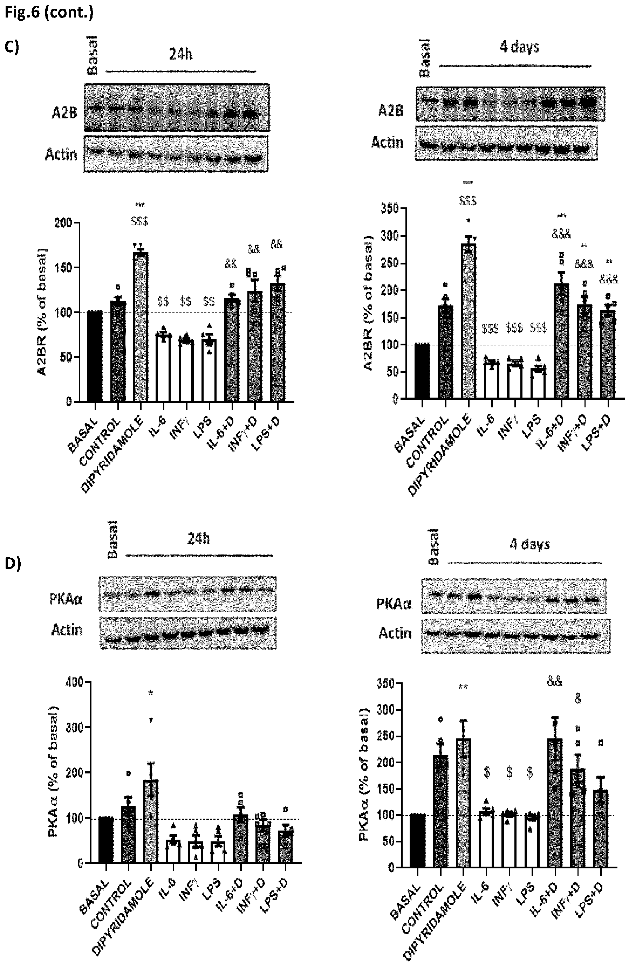

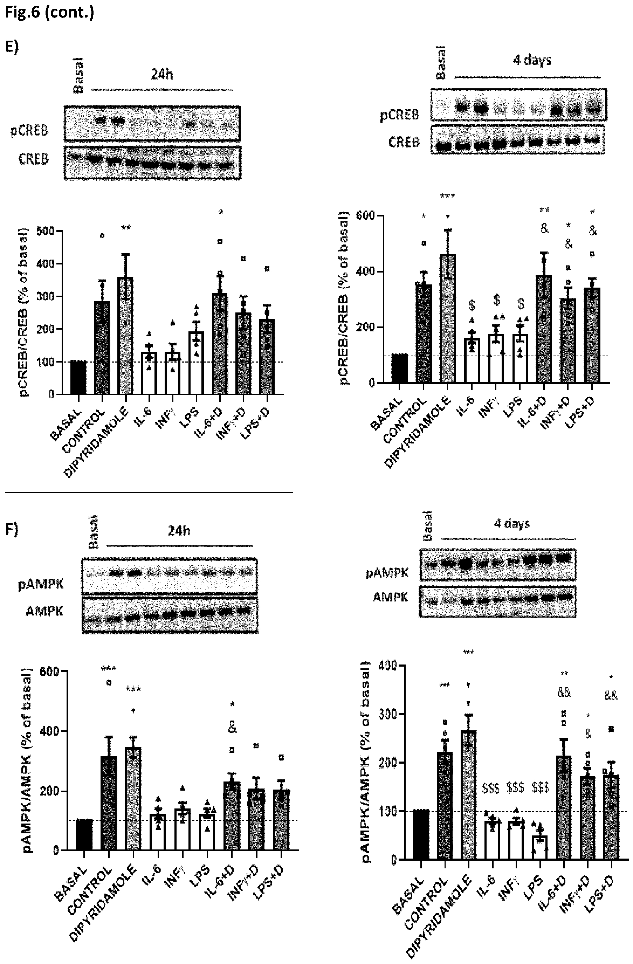

Fig.6: Western blot of C2C12 myoblast cell line in the presence of pro-inflammatory cytokines and dipyridamole at basal, 24h and 4 days of differentiation. Protein expression of (A) Pax7, (B) MHC, (C) A2BR, (D) PKAα, (E) CREB and (F) AMPK. Data are representative of n=5 each group per day (mean ± SEM). ^{∗} Treatment vs basal, $ Treatment vs Control, & pro-inflammatory condition + dipyridamole vs pro-inflammatory condition.

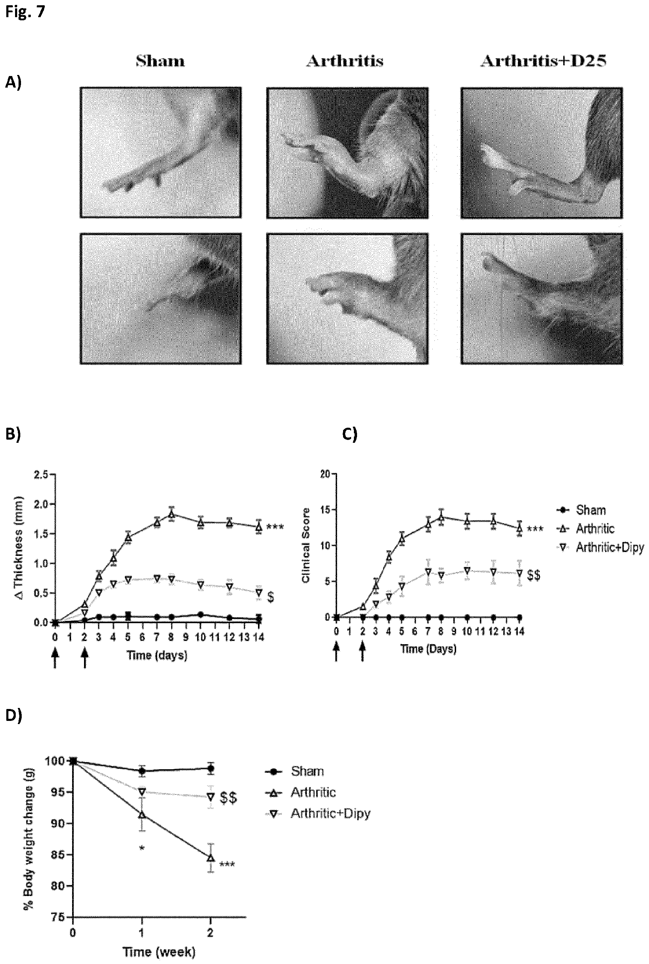

Fig.7: The K/BxN mouse model of RA shows joint pathology and paw inflammation prevented by dipyridamole. (A) Representative image of joints in sham, arthritic and dipyridamole treated mice. (B) Time course of joint width measurements taken throughout the 2-wk study design. (C) Joint clinical score. (D) Body weight measurements represented as the difference in time from the start weight of the trial. Black arrow indicates day of K/BxN serum injection (100uL IP). (* indicates differences with the sham group and $ indicates differences with the arthritic group for B, C and D). Values are presented as mean ± SEM.

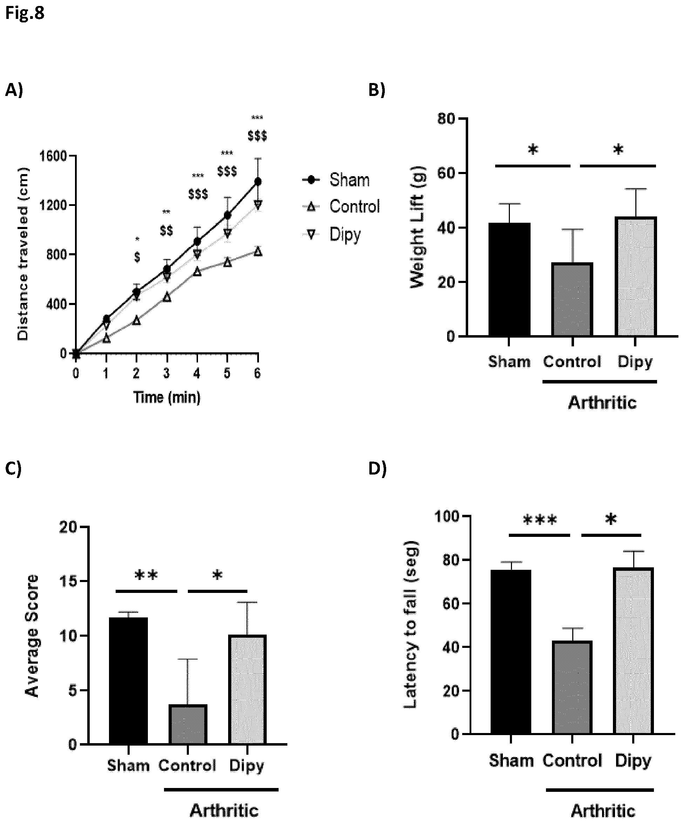

Fig.8: Motor activity tests reflect improvement with the use of dipyridamole in arthritic mice. (A) Ambulation test for 6 min. (B) Weight test with 11g weight rings. (C) Two limb hanging test as a physical endurance test in fall for 3 min. (D) Rotarod test to evaluate motor coordination for 5 min with a speed increase to 40 rpm. (^{∗} indicates differences with the sham group and $ indicates differences with the arthritic group for A; ^{∗} indicates differences between groups for B, C and D). Values are presented as mean ± SEM.

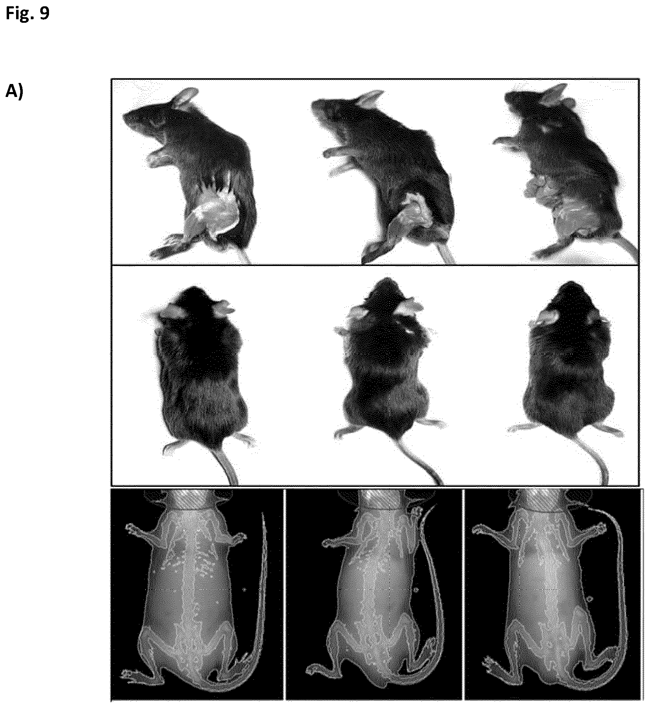

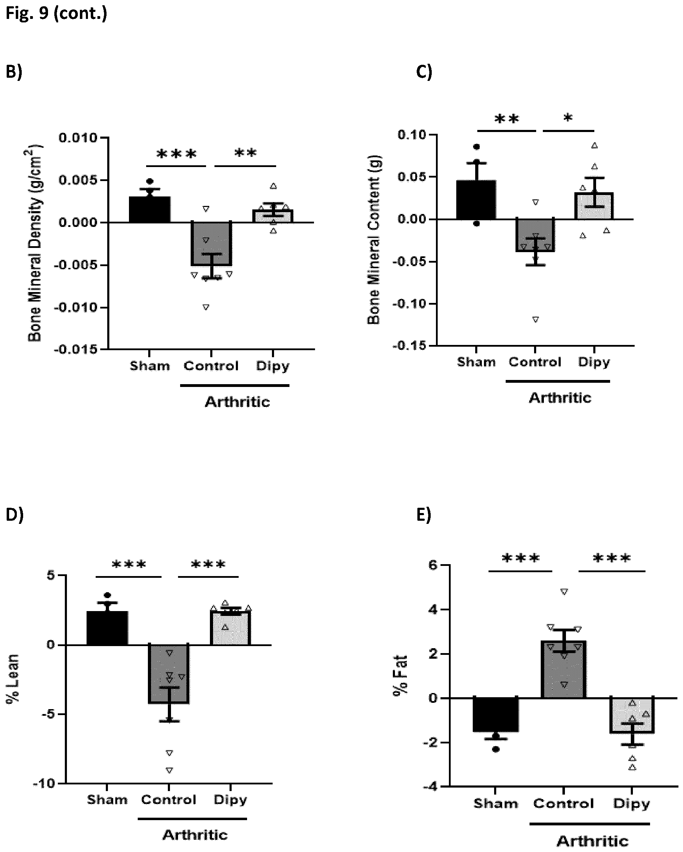

Fig.9: Body composition analysis by DXA scan indicates dipyridamole counteracts the lean tissue, BMD and BMC loss and fat gain in arthritis. (A) Photographic and DXA scan analysis after 2 weeks. Full body DXA scan for (B) BMD, (C) BMC, (D) Lean tissue and (E) Fat expressed as the difference between the value obtained before arthritis induction and after two weeks. ^{∗}indicates differences between groups for B, C, D and E. Values are presented as mean ± SEM.

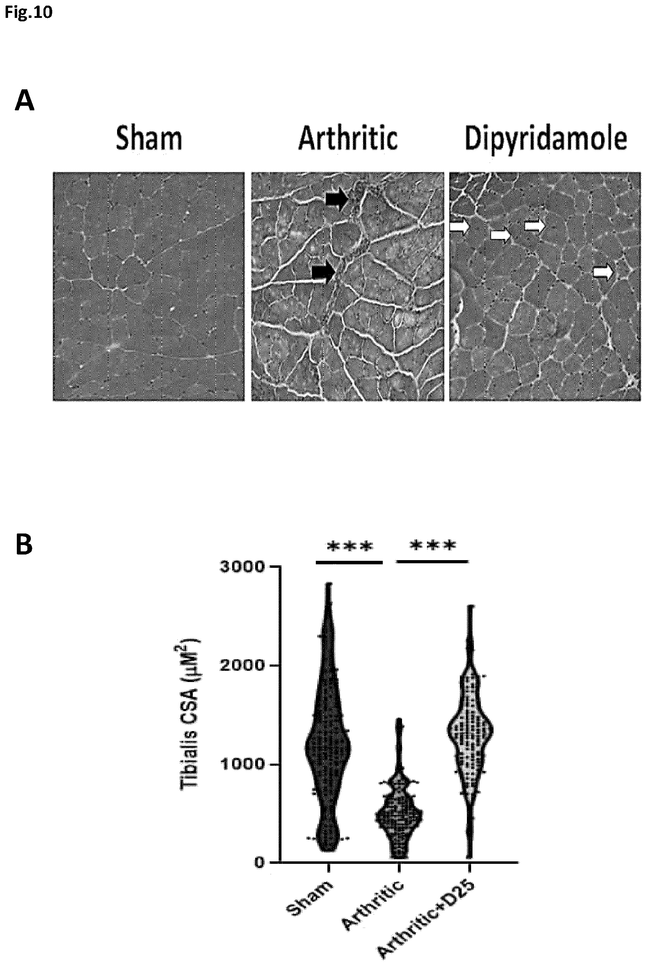

Fig.10: Histological H&E analysis of tibialis indicates that dipyridamole prevents arthritisassociated muscle atrophy. (A) H&E staining of tibialis for sham, arthritic and dipyridamole treated groups. (B) Cross sectional area analysis of H&E-stained muscle fibers by ImagePro V2.2. ^{∗} Indicates differences between groups for B. Values are presented as mean ± SEM.

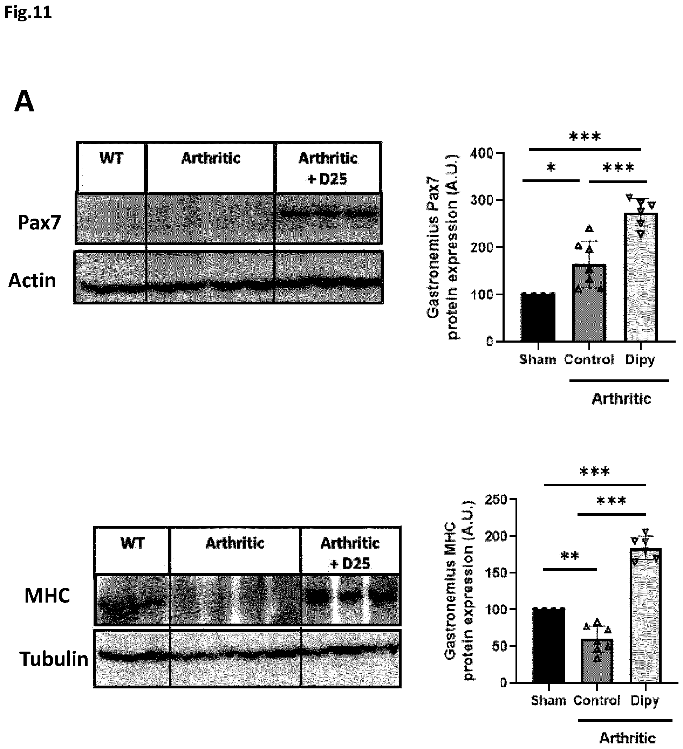

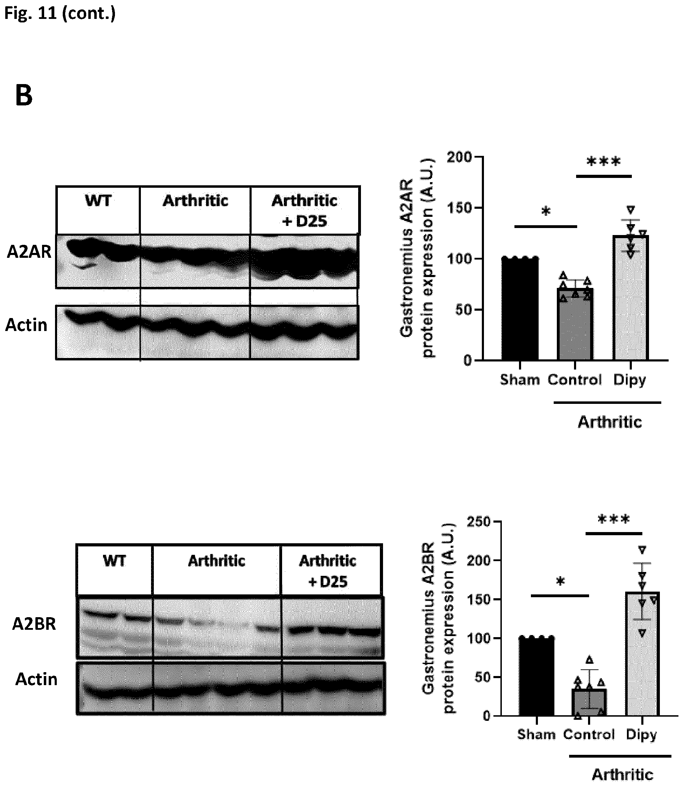

Fig.11: Dipyridamole potentiates the regenerative state in muscle in the arthritic process via A2A and A2B adenosine receptors. Protein expression analyzed by western blot for (A) Pax7, MHC and (B) A2A/A2B adenosine receptors. ^{∗}Indicates differences between groups. Values are presented as mean ± SEM.

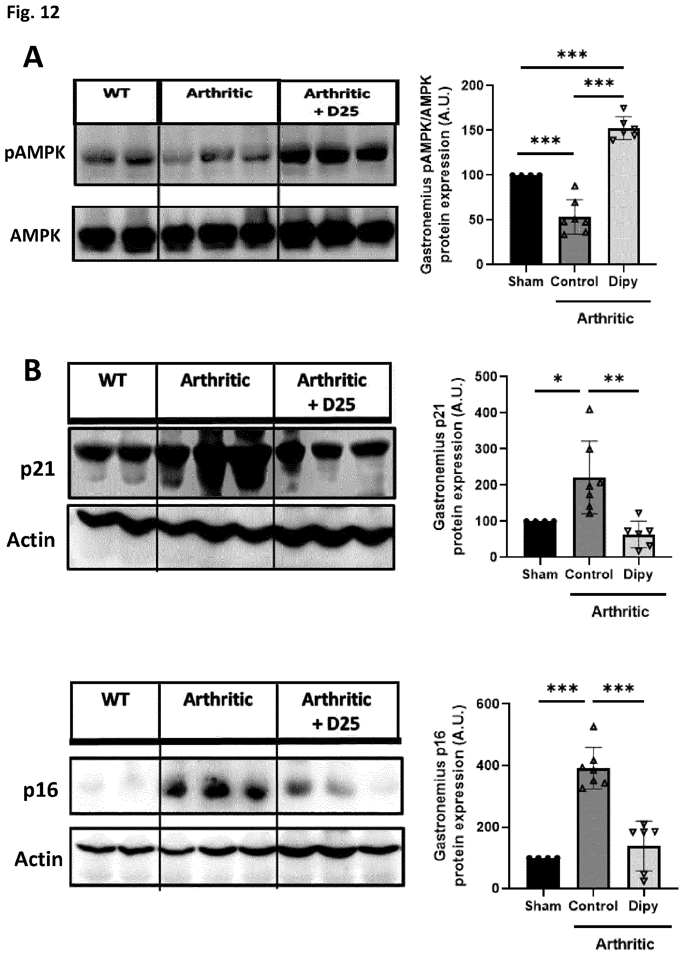

Fig.12: Activation of AMPK and reduction of muscle senescence with dipyridamole treatment in arthritis. Western blot analysis of (A) AMPK expression and (B) senescence proteins p21 and p16. ^{∗} Indicates differences between groups.

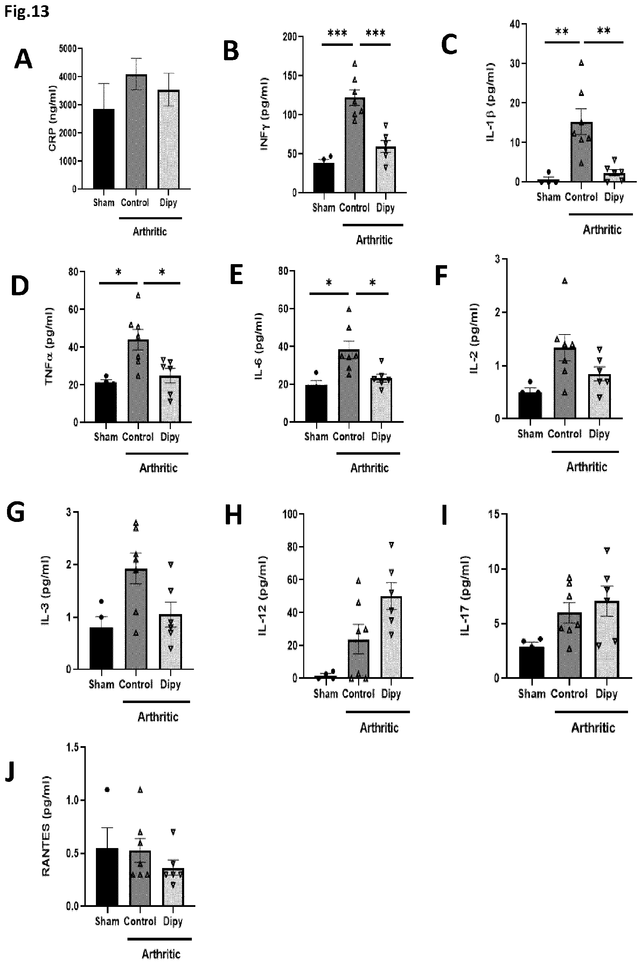

Fig.13: Dipyridamole reduces systemic inflammation associated with arthritis. Study of CRP expression by ELISA in serum. (A) Analysis of protein expression of proinflammatory chemokines and cytokines by fluorescence array for (B) INFγ, (C) IL-1β, (D) TNFα, (E) IL-6, (F) IL-2, (G) IL-3, (H) IL-12, (I) IL-17 and (J) RANTES. ^{∗} Indicates differences between groups. Values are presented as mean ± SEM.

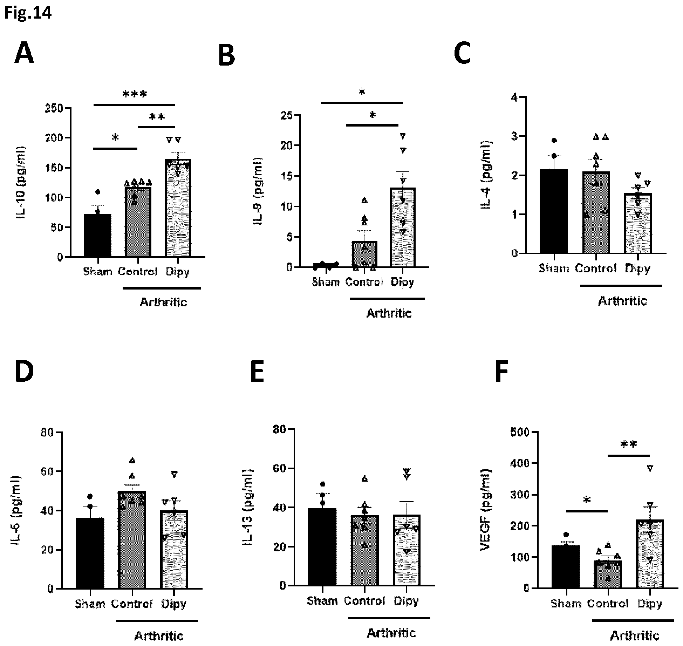

Fig.14: Enhancement of anti-inflammatory response with dipyridamole in the treatment of arthritis. Proteomic array study of anti-inflammatory cytokines and chemokines (A) IL-10, (B) IL-9, (C) IL-4, (D) IL5, (E) IL-13 and the angiogenesis factor (F) VEGF. ^{∗} Indicates differences between groups. Values are presented as mean ± SEM.

### SUMMARY OF THE INVENTION

The present invention relates to dipyridamole, its salt or derivative, for use in a method of treatment, amelioration or prevention of muscular myogenesis disorders or inflammatory arthritis, comprising administering to a subject in need thereof a therapeutically effective amount of dipyridamole, its salt or derivative.

In one embodiment of the present invention the muscular myogenesis disorder is induced by the ageing of the subject.

In one embodiment of the present invention the muscular myogenesis disorder is induced by inflammatory disease(s) present in the subject, preferably chronic inflammatory disease(s).

In one embodiment of present invention the muscular myogenesis disorder is sarcopenia, specifically primary or secondary sarcopenia, preferably wherein said primary or secondary sarcopenia is induced by inflammatory disease(s) present in the subject, preferably chronic inflammatory disease(s).

In one embodiment of present invention the inflammatory arthritis is selected from rheumatoid arthritis (RA) or a type of spondyloarthropathy, such as for example psoriatic arthritis and Systemic Lupus Erythematosus (SLE).

In one embodiment of present invention the inflammatory arthritis is rheumatoid arthritis (RA) or rheumatoid cachexia (RC).

In one embodiment of present invention dipyridamole, its salt or derivative, is administered to the subject in need thereof at between 25 to 100 mg/kg/day, preferably between 50 to 75 mg/kg/day.

In one embodiment of present invention the administration is oral, intravenous, topical, or intramuscular, preferably the administration route is oral.

In another embodiment of present invention dipyridamole is provided in a pharmaceutical composition further comprising pharmaceutically acceptable excipients.

### DETAILED DESCRIPTION OF THE INVENTION

### DEFINITIONS

As used herein the term "dipyridamole" refers to 2,6-bis(Diethanolamino)-4,8-dipiperidinopyrimido[5,4-d] pyrimidine.

As used herein the term "muscular myogenesis disorders" relates to disorders in the formation of skeletal muscular tissue. Herein the term refers specifically to muscular myogenesis disorders that are induced by conditions such as aging and inflammation in the subject.

As used herein the term "sarcopenia" refers to a generalized and progressive loss of skeletal muscle mass and muscle function. Primary sarcopenia is associated with aging, whereas secondary sarcopenia has its causes in other diseases/factors, such as chronic inflammatory disease and/or organ failure.

As used herein the term "inflammatory arthritis" relates to painful inflammation and stiffness of the joints. Arthritis can be broadly classified into two categories, inflammatory arthritis and non-inflammatory arthritis. Inflammatory arthritis can be due to several etiologies, including infectious and non-infectious, and may or may not be associated with systemic features of the underlying condition causing inflammatory arthritis. If left untreated, inflammatory arthritis invariably leads to joint damage and deformities. Types of inflammatory arthritis are for example rheumatoid arthritis and spondyloarthropathy.

As used herein the term "Rheumatoid arthritis (RA)" relates to a chronic, inflammatory and autoimmune disease of unknown etiology that causes destruction of joint cartilage and bone and reduced life expectancy (16).

As used herein the term "Rheumatoid cachexia (RC)" relates to a condition characterized by changes in body composition including reduced fat-free mass, with muscle mass as the major component, and stable or increased fat mass, results in limited changes in body mass index (20,21).

As used herein the term "Psoriatic arthritis" refers to a type of arthritis linked with psoriasis, a chronic skin and nail disease. Psoriasis causes red, scaly rashes and thick, pitted fingernails. Psoriatic arthritis is similar to rheumatoid arthritis (RA) in symptoms and joint swelling (inflammation).

As used herein the term "Spondyloarthropathy (SpA)" refers to the clinical family of disorders that are characterized as inflammatory rheumatic disorders with manifestations in the vertebral column, peripheral joints, and extra-articular structures. Specific types of SpA include psoriatic arthritis and Systemic Lupus Erythematosus (SLE).

The use of the word "a" or "an" may mean "one," but it is also consistent with the meaning of "one or more," "at least one," and "one or more than one". The use of the term "another" may also refer to one or more. The use of the term "or" in the claims is used to mean "and/or" unless explicitly indicated to refer to alternatives only or the alternatives are mutually exclusive.

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps. The term "comprises" also encompasses and expressly discloses the terms "consists of" and "consists essentially of". As used herein, the phrase "consisting essentially of" limits the scope of a claim to the specified materials or steps and those that do not materially affect the basic and novel characteristic(s) of the claimed invention. As used herein, the phrase "consisting of" excludes any element, step, or ingredient not specified in the claim except for, e.g., impurities ordinarily associated with the element or limitation.

As used herein, words of approximation such as, without limitation, "about", "around", "approximately" refers to a condition that when so modified is understood to not necessarily be absolute or perfect but would be considered close enough to those of ordinary skill in the art to warrant designating the condition as being present. The extent to which the description may vary will depend on how great a change can be instituted and still have one of ordinary skilled in the art recognize the modified feature as still having the required characteristics and capabilities of the unmodified feature. In general, but subject to the preceding discussion, a numerical value herein that is modified by a word of approximation such as "about" may vary from the stated value by ±1, 2, 3, 4, 5, 6, 7, 8, 9, or 10%. Accordingly, the term "about" may mean the indicated value ± 5% of its value, preferably the indicated value ± 2% of its value, most preferably the term "about" means exactly the indicated value (± 0%).

The terms "comprise" and "comprising" are used in the inclusive, open sense, meaning that additional elements may be included. The term "comprises" also encompasses and may be used interchangeably with the terms "consists of" and "consists essentially of".

### DETAILED DESCRIPTION

Dipyridamole is able to inhibit nucleoside transport producing an increase of extracellular adenosine levels by inhibition of cellular adenosine uptake. It has been previously shown by the inventors that dipyridamole reverted the bone deleterious effect of tenofovir both *in vitro* and *in vivo.* They observed that mice treated with tenofovir lost nearly 10% of body weight and this was recovered when mice were treated with dipyridamole. However, dipyridamole has to date not been assessed for treating muscular myogenesis disorders, such as sarcopenia. Nor has its effect on different types of inflammatory arthritis been studied.

With an ever-ageing population and the increasing need for novel treatment options for muscular myogenesis disorders, such as sarcopenia, as well as inflammatory arthritis, the inventors have thus set out to assess the effect of dipyridamole on such conditions.

As a first step the inventors could show that the modulation of the purinergic system alters muscular myogenesis. The results suggest that the modulation of ATP output to the extracellular medium by tenofovir promotes premature muscle differentiation, PAX7 reduction in the early stages of cell differentiation, and an early appearance of the late muscle differentiation marker MHC, with no changes in the intermediate differentiation markers. Early muscle development has been shown to lead to depletion of progenitor cells and cessation of muscle growth (50). Von Maltzahn et. al. showed that the Pax7^{-/-} knockout mouse had early differentiation with loss of function of satellite cells during regenerative myogenesis (51). Besides, Liu et. al. showed a reduction in Pax7⁺ marker in SAMP8 murine model of sarcopenia (52). Therefore, the regulation of Pax7/MHC balance is fundamental to the correct myogenesis and a reduction by tenofovir could favor the appearance of sarcopenic markers. As shown in Example 2 and Fig. 1D, dipyridamole can reverse these early differentiation markers, acting as a counter-effector of tenofovir.

The nucleosides/nucleotides modulation by tenofovir and dipyridamole seems to be crucial for the changes observed in muscle myogenesis by these treatments. In general, an increase in both intra- and extracellular nucleotide levels has been observed with muscle differentiation. Increased nucleotide levels had previously been observed to favor differentiation in other models (53,54). Extracellular ATP levels have been shown to maintain muscle homeostasis during myogenesis (55). Reduced levels of available adenosine have been linked to development of muscle atrophy (56). As shown in example 1 and Figure 1B) early differentiation produced by tenofovir *in vitro* leads to a decrease in adenosine and available extracellular ATP, concomitant with the accumulation of intracellular ATP in a very rapid and prolonged manner, and the decrease in intracellular AMP levels in the last days of differentiation. Dipyridamole reverses these nucleotide levels and increases the levels of extracellular adenosine available.

In human skeletal muscle both A2A and A2B adenosine receptors are localized, but not A1 receptor (57). In C2C12 both gene and protein levels for the four adenosine receptors were detected. A1 and A2A play an antagonistic role in myogenesis. A1 increases with cell differentiation while A2A is only detectable when the muscle is in a proliferative state. This correlates with previous studies that had detected more A2A than A1 receptor in C2C12 (58) and supports this proliferative role of A2A and modulator of muscle differentiation of A1. However, the inventors did not see any effect with tenofovir or dipyridamole, suggesting that the modulatory effect of dipyridamole and the available adenosine is mediated by another adenosine receptor. It has been recently demonstrated that the adenosine A2B receptor is the most expressed of all receptors in human skeletal muscle and is related to the maintenance of satellite cells with age, the delay of muscle senescence and the improvement of strength (58). As can be seen in example 3 and Fig. 2 adenosine A2B receptor levels increased with muscle differentiation, which was lost with tenofovir. Dipyridamole is capable of accentuating the increase in adenosine A2B expression and recovering the levels lost by tenofovir. This suggests that the increase in extracellular adenosine available by dipyridamole is captured by the major receptor in the line and this is capable of activation secondary effectors. With differentiation an increase in the expression of the adenosine receptor A3 with myogenesis was found, which was lost with dipyridamole treatment. This receptor is minor compared to the rest and it is suggested that its functionality in muscle is therefore less (58).

It was postulated that the modulation of tenofovir and dipyridamole of intracellular AMP and ATP levels could serve as a key from catabolism to energy anabolism through the activation of AMPK. The inventors have verified that AMPK phosphorylation is progressive with the days of differentiation. Addition of tenofovir lead to a loss in the AMP/ATP ratio, which is capable of inhibiting AMPK in both myoblast and myofiber. Dipyridamole recovered AMP/ATP levels, maintaining AMPK activation in both models (see example 5 and Fig.4). Emerging studies indicate that the responsiveness of AMPK signaling clearly declines with aging (59). All this suggests a catabolic role for tenofovir and an anabolic role for dipyridamole (60).

Adenosine receptors play an antagonistic role in the modulation of cAMP. The activation of A1/A3 receptors inhibits cAMP and the activation of A2A and A2B receptors promotes cAMP (61). Response-element binding-protein (CREB) phosphorylation due to increased cAMP and subsequent PKA (Protein Kinase A) activation has been related to increased proliferation in muscle myogenesis, hypertrophy/cell migration and regeneration of damaged muscle (62-64). Therefore, elucidating the role of tenofovir and dipyridamole in the activation of cAMP and its effectors was essential to understand their modulator role in myogenesis. *In vitro,* the inventors observed that tenofovir did not modulate cAMP levels, which is explained by the reduction of available extracellular adenosine (see example 4 and Fig.3). Dipyridamole reverses this effect by producing cAMP activation in myoblast and once differentiated in myotube. cAMP has recently been proposed as a molecule that improves motor activity as well as delays the effects of aging (65). It has been observed by Wang et. al. in with the daily treatment with cAMP in 24-month-old mice leads to a delay in the appearance of the age-related phenotype as well as an improvement in motor activity (65). On the other hand, inhibition of adenosine A2B receptor expression led to a decrease in cAMP not seen with adenosine A2A receptor inhibition in vitro (58). The use of inhibitors of phosphodiesterase 4 (PDE 4), the major cAMP-modifying PDE found in skeletal muscle, reduce the loss of muscle mass and force resulting from denervation in rats and mice models (66). All this suggests that cAMP prevents muscle aging and dipyridamole may contribute to muscle maintenance in aging.

Activation of cAMP with dipyridamole leads to increased PKAα expression and CREB phosphorylation. The CREB transcription factor, a final effector of cyclic AMP signaling, has been related to increased myoblast proliferation as well as expression of early myogenic transcription factors in cultured primary myocytes (64). This correlates CREB activation with muscle regeneration after damage (64). Therefore, we can predict that the increase of pCREB via cAMP by dipyridamole could improve the proliferative capacity of myoblast cells during muscle regeneration.

From this data it was concluded that adenosine and ATP act as mediators in muscle myogenesis. Modulation of the purinergic system with compounds that increase extracellular adenosine levels, such as dipyridamole, produces an activation of the A2B adenosine receptor and an increase in cAMP and AMPK signaling. These pathways, known for their anti-aging character in muscle, support the role of dipyridamole as a good therapeutically approach in treating muscle myogenesis disorders, such as for example sarcopenia.

Sarcopenia and frailty have become major problems for our aging society. The Aging in Motion (AIM) Coalition describes sarcopenia as a type of persistent muscle atrophy characterized by gradual loss of skeletal muscle mass and function (strength) with a risk of negative outcomes such as physical disability, poor quality of life and death. Therefore, understanding myogenesis, muscle loss, muscle degeneration and regeneration and effective therapies for muscle diseases including sarcopenia are very important.

Even though sarcopenia is mainly associated with the aging process seen in the elderly (primary sarcopenia), there are several other populations at risk due to lifestyle decisions or pathological states. Secondary sarcopenia can be considered when one or more of the following causes are evident: sedentary, immobilization, malnutrition, diabetes, obesity, and other acute or chronic inflammatory diseases (e.g. Rheumatoid sarcopenia) that could promote the loss of muscle mass.

Sarcopenia can be observed in chronic debilitating diseases, as well as in typical inflammatory conditions, but in these cases, the pattern of sarcopenia depends on the severity of the injury.

The inventors have furthermore set out to confirm the role of dipyridamole in the treatment of inflammatory arthritis types, such as rheumatoid arthritis (RA) and rheumatoid cachexia (RC).

To confirm the findings of the positive effect of dipyridamole seen *in vitro* on the muscular myogenesis disorders *in vivo* and at the same time assess its effect on inflammatory arthritis, the inventors generated arthritic K/BxN mice by crossing KRN mice with NOD/Lt mice as further detailed in Example 8 and performed motor activity tests prior to serum inoculation and at the highest point of inflammation (see Example 9).

The inventors demonstrated that arthritic mice developed sarcopenia accompanied with a worse motor activity, loss of BMD and BMD, an increase in fat, an increased in muscle senescence and an inactivation of AMPK. As expected, arthritic animals had systemic inflammation.

The use of dipyridamole counteracted all these effects. It can reduce arthritic clinical score, preventing joint inflammation. It decreases systemic inflammation by decreasing proinflammatory cytokines and enhancing anti-inflammatory cytokines. The immunomodulatory capacity of adenosine has been known since the 1970s, when its role in the development and activity of several immune cells was first established. Inflammation produces an increase in extracellular adenosine, with levels reaching micromolar range (67). It has been described that increased adenosine and activation of the adenosine A2A receptor inhibits both TNF-α and IL-6 secretion under inflammatory conditions, with an increase in IL10 (68) leading to anti-inflammatory effects mediated by adenosine, as occurs in RA (69).

It has been further demonstrated by the inventors that dipyridamole treatment prevents muscle loss by activated muscle remodeling with increased PAX7 and MHC and activation of AMPK activity, and thereby maintaining the physical activity in arthritic mice. In their model, the inventors observed an increased expression in both MHC and Pax7 in the presence of dipyridamole. Together with an increase in fiber area, a central nuclei location and the decrease in senescence marker, it is hypothesized that dipyridamole is promoting muscle regeneration and active proliferation (70-72).

In the animal model it was shown that Dipyridamole increased both adenosine A2A and A2B receptors. In C2C12 cell lines both gene and protein levels for the four adenosine receptors were detected. Adenosine A2A receptor was only detectable when the muscle was in a proliferative state. This correlates with previous studies that had detected more A2A than A1 receptor in C2C12 (73) and supports the proliferative role of A2A.

In conclusion, arthritic mice developed sarcopenia accompanied by a loss of motor activity, strong systemic inflammation and increased muscle senescence. Dipyridamole counteracted both systemic and joint inflammation as well as muscle loss, enhancing muscle regeneration. This indicates that the use of agents that increase extracellular adenosine levels, such as dipyridamole, can be useful as a therapeutically approach for sarcopenia.

The present invention therefore in one aspect relates to dipyridamole, its salt or derivative, for use in a method of treatment, amelioration or prevention of muscular myogenesis disorders orinflammatory arthritis, comprising administering to a subject in need thereof a therapeutically effective amount of dipyridamole, its salt or derivative.

The subject can be an animal or a human, preferably the subject is a human.

In one embodiment of the present invention the muscular myogenesis disorder is induced by the ageing of the subject. Ageing of the subject refers to the natural process of changes in the subject over time.

In one embodiment of the present invention the muscular myogenesis disorder is induced by inflammatory disease(s) present in the subject, preferably chronic inflammatory disease(s). The inflammatory diseases can therefore be the cause of the induced myogenesis disorder which appears in the subject after a period of time in which the subject has been suffering from such inflammatory disease(s).

In one embodiment of present invention the muscular myogenesis disorder is sarcopenia, specifically primary or secondary sarcopenia, preferably wherein said primary or secondary sarcopenia is induced by inflammatory diseases present in the subject, preferably chronic inflammatory diseases.

In one embodiment of present invention the inflammatory arthritis is selected from rheumatoid arthritis (RA) or a type of spondyloarthropathy, such as for example psoriatic arthritis and Systemic Lupus Erythematosus (SLE).

In one embodiment of present invention the inflammatory arthritis is rheumatoid arthritis (RA) or rheumatoid cachexia (RC).

In one embodiment of present invention dipyridamole, its salt or derivative, is administered to the subject in need thereof at between 25 to 100 mg/kg/day, preferably between 50 to 75 mg/kg/day.

In one embodiment of present invention the administration is oral, intravenous, topical, or intramuscular, preferably the administration route is oral.

In another embodiment of present invention dipyridamole is provided in a pharmaceutical composition further comprising pharmaceutically acceptable excipients.

In a further aspect present invention relates to an inhibitor of adenosine transport for use in a method of treatment, amelioration or prevention of muscular myogenesis disorders or inflammatory arthritis, comprising administering to a subject in need thereof a therapeutically effective amount of said inhibitor of adenosine transport, its salt or derivative.

Said an inhibitor of adenosine transport can be any compounds that is capable of inhibiting the adenosine transport in a subject upon administration thereof. Preferred inhibitors are ticagrelor, and clopidogrel.

Clopidogrel is a thienopyridine that undergoes metabolic transformation in the liver to generate an active metabolite that binds irreversibly to P2Y12and thereby inhibits ADP-induced platelet activation.

Ticagrelor is a more recently developed antiplatelet agent that, in addition to P2Y12 inhibition, prevents cellular adenosine uptake by the equilibrating nucleoside transporter (ENT)-1 and thereby increases extracellular adenosine levels.

Both antiplatelet agents ticagrelor and clopidogrel (and its active metabolite CAM) regulate osteoclast differentiation by different mechanisms and that ticagrelor, like dipyridamole, inhibits osteoclast differentiation by an A2AR-mediated mechanism (74).

The following examples are provided to illustrate the invention and are not to be construed as limiting the scope of the invention.

### EXAMPLES

### MATERIALS AND METHODS

### Cell culture

C2C12 cells (American Type Culture Collection, ATCC, Manassas, VA, USA) were cultured in DMEM supplemented with 10% FBS and 1% penicillin-streptomycin. To induce myogenic differentiation, 70% confluence C2C12 cells were culture in DMEM containing 2% HS (horse serum). Cells were maintained in a humidified chamber at 37°C, in 95% air and 5% CO2. Media was replaced with fresh every 48h.

### Cell proliferation assay

To assess the suitable doses for the present studies, the inventors performed a dose response proliferation assay using the previously administered dosages of 1µM dipyridamole which was shown to revert the bony deleterious effects of 1µM tenofovir.C2C12 cells were plated in 96-well plates (Corning, Cultek, Spain) (12.500 cells/well). Cell proliferation was measured using AlamarBlue assay according to the manufacturer's instructions, at 24 and 48h of myogenic differentiation. Cells were treated with tenofovir or dipyridamole at 10⁻⁹-10⁻⁵ M.24h after 2% HS addition, tenofovir showed a dose-response inhibition in proliferation compared to control (63.3±5% tenofovir 1 µM and 62.8±6.4% tenofovir 10 µM vs. 100±7.3% control, *p<0.05*) that was increased at 48h. No significant changes were observed at any dose in presence of dipyridamole both 24 and 48 hours of incubation. Therefore, 1µM was used for both compounds for all experiments as described here below.

### RNA isolation and quantitative real time polymerase chain reaction (RT-PCR)

C2C12 were treated with tenofovir, dipyridamole or a combination of both (1 µM each) in 2% HS for 4 days. Total RNA was extracting using TRIzol reagent and retrotranscribed using MuLV Reverse transcriptase PCR kit (Applied Biosystems, Foster City, CA, USA). Relative quantification of gene expression was performed using real-time RT-PCR on a Step One Plus (Applied Biosystems) with Power UP SYBR Green MasterMix according to the manufacturer's protocol. Primers on Table 1 were used. The Pfaffl method was used for relative quantification ²².

### Western Blot

C2C12 were treated with tenofovir, dipyridamole or a combination of both (1 µM each) with/without PSB-603 (1 µM) in 2% HS for up to 4 days. Cells were lysed with RIPA buffer and protein concentration was determined by BCA. 4 µg of protein was subjected to 6-15% SDS-PAGE and transferred to a nitrocellulose membrane. Membranes were blocked with 3% BSA in Tris-buffered saline containing 0.1% Tween-20 (TBS-T). Membranes were incubated overnight at 4°C with the primary antibodies for adenosine A1, Panx-1 (Proteintech, Rosemont, USA), A2A, A2B, A3, MHC (Thermo Fisher Scientific, Waltham, USA), P2X7 (Abcam, Cambridge, UK); AMPK, pAMPK (Thr172), PKAα, PKAβ, pCREB (Ser133), CREB, PKAγ (Cell signaling, Danvers, EEUU) (1:1000 each) and PAX-7 (Developmental Studies Hybridoma Bank, Houston, EEUU) (1:500). After washed in TBS-T and incubated with the secondary antibody anti-rabbit IgG HRP or anti-mouse IgG HRP (1:1000 each) (Thermo Fisher Scientific) for 1h, immune complexes were visualized using immobilon HRP substrate (Millipore, Danvers, USA) and adquired with Amersham Imager 600 (GE Healthcare Life Sciences). Reprobing with actin was performed to check that all lanes were loaded with the same amount of protein. Digital densitometric band analysis was performed using the Quantity One software (Bio-Rad, Madrid, Spain) and band intensities were expressed relative to actin. Variations in intensity were expressed as % of basal (day 0) and expressed as mean ± SEM. All results were calculated as a percentage of nondifferentiated controls to minimize the intrinsic variation among different experiments.

### cAMP concentration

C2C12 were seeded at 200.000 cells/well and at day 0 and 4 of differentiation, cells were treated with tenofovir and dipyridamole 1 µMeach for up to 2 hours. cAMP concentration was measured using the colorimetric cAMP ELISA kit following manufacture protocol for acetylated cell lysates (Cell Biolabs, San Diego, USA). Absorbance was measured at 450 nm using a microplate reader Tecan Spark 20M.

### AMPK activity

C2C12 were sedeed at 12.500 cells/well in a 96 well black (clear bottom) plate and at day 0 and 4 of differentiation, cells were treated with tenofovir and dipyridamole 1 µM each for up to 4 hours. Assay was performed wirh the AMPK phosphorilation assay kit (Abnova Corporation, Jhouzih St, Taiwan) as manufactor instructions and pAMPK was mesured at λex/em 530/585nm and 360/450 nm for total protein with Tecan Infinite.

### Determination of nucleotides concentration by HPLC

C2C12 were treated with tenofovir, dipyridamole or a combination of both (1 µM each) in 2% HS for up to 4 days. Supernatant and cell lysate are collected every day. Cells were lysated with a homemade buffer (1mM phenylmethylsulfonyl fluoride, 1mM protease inhibitor cocktail, 1mM sodium orthovanadate, 1mM sodium fluoride and 1mM *β*-glicerophospate) and supernatant was used directly. A heat shock step was done at 98ºC for 2 min, and to avoid adenosine degradation 1 µM EHNA was added at the time of heating. All samples were centrifuged at 13000g for 10 min at 4°C and supernatants were collected and stored at -80°C until use. Inosine, adenosine, AMP, ADP and ATP concentrations were determined with high performance liquid chromatography (HPLC) using a liquid chromatograph with a reversed phase column (Agilent 1100 Series Liquid Chromatography) and an UV detector set at 254nm. Buffer composed of 0.1 mol/L KH₂PO₄ (pH 7.5) and 18% acetonitrile was run at 1.5 ml/min for 20 min. Compounds were identified and quantified by their retention times and peak areas of known standards, calibrated by spectrophotometry. Heat shock process induced a loss of adenosine (6.31% loss), ADP (1.32% loss) and ATP (0.44% loss) (standard not heat shock vs standard heat shock) that was taken in consideration for analysis (Figure 1A). The results have been corrected and expressed as mean ± SEM. All results were calculated as a percentage of controls without differentiation state.

### Statistical analysis

The statistical significance of differences among groups was determined by the use of one-way ANOVA and Bonferroni post hoc test. All statistics were calculated using GraphPad software (La Jolla, CA, USA).

### Example 1: Tenofovir and Dipyridamole modulate adenosine nucleotide concentrations and muscle differentiation markers

To understand if dipyridamole or tenofovir treatment induced changes in adenosine nucleotides concentrations sufficiently to modulate myogenesis, the concentration was analyzed by HPLC as described in the materials and methods part above.

At the extracellular level, inosine was neither changed within differentiation nor by the respective treatment as shown in Fig 1. Extracellular adenosine increased within differentiation (11.6±0.81nM control at day 4 vs 5.65±0.88nM basal, *p<0.001*), been significant also at day 2 (*p<0.005*) (Fig 1B). Tenofovir decreased extracellular adenosine concentrations within differentiation (6.65±0.54nM tenofovir vs 11.6 ± 0.81nM control at day 4, *p<0.001*) been significant at day 1 and 2. Dipyridamole treatment increased the levels of extracellular adenosine (*p<0.001*), being significant compared to control at day 3 and 4 (17.33±0.47nM dipyridamole vs 11.6±0.81nM control at day 4, *p<0.001*) (Fig 1B), and been significant compared to tenofovir from day 1 to 4 (*p<0.001*) as the combined treatment increased extracellular adenosine levels similar to dipyridamole alone (Fig 1B). No measurable levels of extracellular AMP were detected in the supernatant. In the presence of 2% HS, ADP levels remained constant until day 2 and increased from day 3 (*p<0.001*) of differentiation (5.88±0.18nM control at day 4 vs 1.09±0.49nM basal, *p<0.001,* Fig 1B) and were not modulated by treatments (*p*=*ns*). Extracellular ATP levels increased within differentiation (14.7±0.5nM control at day 4 vs. 3.02±0.3nM basal, *p<0.001*), been significant at day 2 (*p<0.001*) and 3 (*p<0.001*) (Fig 1B). Tenofovir reduced extracellular ATP levels at day 4 (10.67±0.28nM tenofovir at day 4 vs. 14.7±0.5pM control at day 4, *p*=*0.005,* Fig 1B). Dipyridamole did not modify extracellular ATP levels compared to control but reverted the effect of tenofovir when both combined (13.21±0.22nM T+D vs. 10.67± 0.28nM tenofovir at day 4, *p<0.001,* Fig 1B).

When we analyzed intracellular nucleotides levels, we observed that 2% HS increased inosine levels (4.98±0.41nM control at day 4 vs. 2.27±0.04nM basal, *p<0.001*), been significant at day 2 (*p<0.001*) (Fig 1C). Tenofovir did not modulate these levels (*p*=*ns*), meanwhile dipyridamole increased inosine levels at day 4 (14.17±1.75nM dipyridamole vs. 11.07±0.54nM control, *p<0.001,* Fig 1C), but it was not able to revert tenofovir effect (*p*=*ns*). Intracellular adenosine increased progressively during the 4 days of differentiation (10.87±0.73nM control at day 4 vs 2.10±0.19nM basal, *p<0.001,* Fig 1C). Tenofovir did not change intracellular adenosine levels compared to control (*p*=*ns*), but dipyridamole increased them at day 4 of differentiation (13.76±1.13nM dipyridamole vs. 10.87±0.73nM control at day 4, *p<0.001*) and reverted tenofovir effect (*p*=*0.001,* Fig 1C). Intracellular AMP levels increased within differentiation (12.10±0.73nM control at day 4 vs. 3.33±0.18nM basal, *p<0.001*), been significant from day 1 (*p<0.001*) (Fig 1C). Tenofovir reduced AMP levels compared to control been significant at days 3 and 4 (6.33±0.35nM tenofovir vs. 12.1±0.73pM control at day 4, *p<0.001,* Fig 1C). Dipyridamole increased intracellular AMP (18.53±1.13nM dipyridamole vs. 12.10±0.73nM control at day 4, *p<0.001*), been significant at day 1 to 3 (*p<0.001*) and reverted tenofovir effect (p<0.001) (Fig 1C). ADP levels increased on day 1 in the presence of 2% HS (2.86±0.49nM control vs 0.87±0.04nM basal, *p<0.001*) and treatments did not modulate the levels (Fig 1C). Intracellular ATP levels increased with 2% HS at day 2 (7.44±0.73nM control vs 1.94±0.35nM basal, *p<0.001*), and remained constant until day 4 (*p<0.001,* Fig 1C). Tenofovir increased intracellular ATP starting at day 1 of differentiation compared to the control (11.56±0.26nM tenofovir vs 4.93±0.02nM control, *p<0.001,* Fig 1C). Dipyridamole maintained intracellular ATP levels similar to control and reverted the effect of tenofovir (4.13±0.44nM T+D vs 11.74±0.08nM tenofovir at day 1, *p<0.005,* Fig 1C).

### Example 2: Myogenesis markers

Next, myogenesis markers were studied to analyze if the observed changes in intra/extracellular nucleotides were able to modulate them. As expected, PAX7 increased the first day of induction with 2% HS (315±58% control at day 1 vs basal, *p<0.05,* Fig 1D) been reduced to basal levels within differentiation. Tenofovir decreased PAX7 expression to basal levels (101±23% tenofovir vs 315±58% control at day 1, *p<0.05,* Fig 1D) and dipyridamole was able to revert the effect to control levels (*p<0.005,* Fig 1D). Myf5 levels remained stable during differentiation (*p*=*ns*) with tenofovir increasing them during the first 2 days of differentiation (142±23% tenofovir vs basal, *p<0.001,* Fig 1D). Dipyridamole did not modulate Myf5 expression (*p*=*ns*). MyoD protein expression showed a progressive increase within differentiation (255±20% control at day 4 vs basal, *p<0.005,* Fig 1D) that was enhanced with tenofovir (389±33% tenofovir vs 182±8% control at day 2, *p<0.001,* Fig 1D) and been reverted by dipyridamole (162±29% dipyridamole vs. 389±33% tenofovir, *p<0.001,* Fig 1D). MyoG expression increased with 2% HS (*p*=*ns*) but was not modulated with the use of treatments (*p*=*ns*). MHC was expressed at the end of cell differentiation as expected (839±57% control at day 4 vs basal, *p<0.05,* Fig 1D). Tenofovir led to a premature expression of MHC (1429±379% tenofovir vs 839±57% control at day 1, *p<0.05,* Fig 1D), been dipyridamole able to revert the effect (175±76% T+D vs 1153±131% tenofovir, *p<0.05,* Fig 1D).

### Example 3: Purinergic receptors are modulated within myoblast differentiation

The expression of adenosine receptors, P2X7 receptor and Panx-1 channel was analyzed during myoblast differentiation. A1 receptor protein expression increased significantly during differentiation (699±105% control vs basal, *p<0.005,* Fig 2) with no modulation by treatments (*p*=*ns*). Adenosine A2A receptor protein expression decreased with differentiation (34.9±10.8% control at day 4 vs basal, *p<0.005,* Fig 2) been significant from day 1 (*p<0.005*), and treatments did not modulate the expression (*p*=*ns*)*.* Adenosine A2B receptor protein expression increased with 2% HS (652±148% control at day 4 vs basal, *p<0.05,* Fig 2). Tenofovir did not modulate A2B expression compared to basal levels (Fig 2), and dipyridamole increased A2B protein expression and reverted the effect of tenofovir (884±132% T+D vs 303±60% control and 553±47% tenofovir at day 4, *p<0.05* and *p<0.001* respectively, Fig 2). A3 receptor protein expression increased with 2% HS (689±68% control at day 4 vs basal, *p<0.001,* Fig 2), been significant at day 2 and 3 (*p<0.001*). Tenofovir did not change A3 expression when compared to control as well as dipyridamole alone or in combination (*p*=*ns*) (Fig 2). 2% HS increased Panx-1 protein expression (1567±30% control at day 4 vs basal, *p<0.001,* Fig 2) but this was not changed with treatments (*p*=*ns*). Finally, 2% HS did not change P2X7 protein expression during differentiation (*p*=*ns*) and tenofovir did not module P2X7 expression (*p*=*ns,* Fig 2). However, dipyridamole increased P2X7 expression compared to tenofovir (202±46% dipyridamole vs 89±19% tenofovir at day 4, *p<0.05,* Fig 2), but it was not able to revert the effect of tenofovir (*p*=*ns*).

### Example 4: Dipyridamole increased cAMP and activates the PKA pathway

The cAMP signaling cascade has been linked to a delay in the onset of age-related muscle loss. When we analyzed cAMP activation at early time points, we observed an increase in cAMP levels 5 minutes after 2% HS incubation (0.58±0.13 control vs basal, *p=0.005,* Fig 3A) that progressively decreased with time. Dipyridamole increased cAMP at 5 minutes that was maintained up to 15 minutes (0.85± 0.17 dipyridamole vs 0.58± 0.13 control at 5 minutes, *p=0.005,* Fig 3A) and tenofovir reduced cAMP levels (0.24± 0.07 tenofovir vs 0.58± 0.13 control at 5 minutes, *p=0.005,* Fig 3A). The combine treatments (both dipyridamole and tenofovir pretreatments) had similar cAMP values as dipyridamole alone (Fig 3A). Similar results were obtained when C2C12 were differentiated to myofiber for 4 days and then treated with tenofovir and dipyridamole (Fig 3A).

To study the cAMP signaling pathway, we analyzed the expression of PKA on its three catalytic isoforms PKAα,PKAβ and PKAγ as well as EPAC1/2. PKAα increased within differentiation (292±65% control at day 4 vs basal, *p=0.05,* Fig 3B), and tenofovir reduced this expression (215±90% tenofovir vs basal, *p=ns,* Fig 3B), been dipyridamole able to revert this effect (429±99.4% T+D vs 215.8±94.2% tenofovir at day 4 of differentiation, *p<0.05*) in a similar manner as dipyridamole alone (Fig 3B). PKAβ and PKAγ expression increased within differentiation, but no changes were observed with any treatment (Fig 3B). pCREB increased within differentiation (247±30% control at day 4 vs basal, *p=0.05,* Fig 3B). Tenofovir reduced its phosphorilation (154±9% tenofovir vs 247± 30% control at day 4, *p<0.05,* Fig 3B). Dipyridamole increased pCREB similar to control (275±73% of dipyridamole at day 4 vs basal, *p=0.05,* Fig 3B) and reverted tenofovir effect (*p<0.05,* Fig 3B). We observed no changes in EPAC1 but a decreased on EPAC2 within differentiation with no differences among treatments (Fig 3B), meanwhile EPAC2 was decreased with 2% HS and dipyridamole, and tenofovir maintained the expression similar to basal levels (Fig 3B).

### Example 5: Dipyridamole improves AMPK activation

As AMPK activation depends on the intracellular AMP/ATP ratio, we established the AMP/ATP ratio according to HPLC values. AMP/ATP ratio remains stable during the 4 days of differentiation with 2% HS when compared to basal (*p=ns*) and it was significantly reduced with tenofovir (0.62± 0.03 tenofovir vs 1.88± 0.12 control at day 3, *p<0.001,* Fig 4A). Dipyridamole reverted tenofovir effect to control levels (2.19± 0.17 T+D vs 0.62± 0.03 tenofovir, *p<0.05,* Fig 4A) resulting in an AMP/ATP ratio similar to dipyridamole alone.

We next studied if these changes in AMP/ATP ratio had any effect in AMPK activation. Western Blot analyses revealed no differences in pAMPK at Tyr172 among the 4 days of differentiation in 2% HS (Fig 4B) that were not modulated with tenofovir (1.15±0.05 tenofovir at day 4 vs basal, *p=ns,* Fig 4B) and dipyridamole increased pAMPK at day 4 of differentiation when compared to control (2.66±0.7% dipyridamole vs 1.29± 0.27% control at day 4 of differentiation, p<0.01, Fig 4B). The combined treatment increased pAMPK at day 4 when compared to tenofovir (2.58±0.73% T+D vs 1.15±0.05 tenofovir at day 4 of differentiation, p<0.05, Fig 4B).

These results tend to indicate that at long term, the decrease AMP/ATP ratio with tenofovir did not alter AMPK activation. To prove AMPK activation at short time points, AMPK activation assays both at myoblast and myofiber stages was performed. When we analyzed AMPK activation in myoblast, AMPK activation increased 10 min after switching to 2% HS (1.95±0.34% control vs basal, p<0.05, Fig 4C), with tenofovir keeping AMPK inactive (1.02±0.19 tenofovir vs 1.97±0.17 control, *p=0.05,* Fig 4C). Dipyridamole increased these levels at 5 and 10 minutes (2.89±0.29 dipyridamole vs 1.97±0.17 control, *p<0.05,* Fig 4C), and reverted the inactivation of AMPK induced by tenofovir (Fig 4C). Similar results were obtained when C2C12 were differentiated to myofiber for 4 days and then treated with tenofovir and dipyridamole for short time (Fig 4D).

### Example 6: Adenosine A2B receptor blockade reverses dipyridamole myogenesis modulation

The results shown in this manuscript indicate that dipyridamole was exerting its effect by activation of the A2B receptor. To prove it, the inventors treated the cells with the selective A2B inhibitor PSB-603. PSB-603 completely reverted the increased in PAX7 induced by dipyridamole (83±18% dipyridamole+PSB-603 vs 198±24% dipyridamole at day 1, *p<0.005)* in a similar manner as the inhibitor alone (Fig 5A). PSB-603 also reverted the decreased in Myf5 induced by dipyridamole (580± 76.4% dipyridamole+PSB-603 vs 79.48± 31% dipyridamole at day 3, *p<0.001,* Fig 5A). PSB-603 pre-treatment led to the early expression of MHC (408±78% dipyridamole+PSB-603 vs 95±13% dipyridamole at day 1, *p<0.005*) in a similar manner as PSB-603 alone (Fig 5A).

PSB-603 pre-treatment decreased PKAα expression when compared to dipyridamole (59±26% dipyridamole+PSB-603 vs 358±156% dipyridamole at day 4, *p<0.05,* Fig 5B) with a concomitant decreased in pCREB (79.6± 22.1% dipyridamole+PSB-603 vs 399.6±119.8% dipyridamole at day 4, *p<0.005,* Fig 5B). PSB-603 also decreased pAMPK expression (74.1±33% dipyridamole+PSB-603 vs 284±79.8% dipyridamole at day 4, *p<0.001,* Fig 5C).

### Example 7: Dipyridamole prevents inflammatory aberrant proliferation via A2B and PKA/AMPK

C2C12 were treated with IL-6 (2.5µg/ml), INFγ (1µg/ml) or LPS (10µg/ml) in 2% HS for up to 4 days in the presence or absence of dipyridamole 1µM. Western blot was performed as described above.

To verify the myogenesis-correcting effect of dipyridamole observed in the presence of tenofovir, the expression of muscle proliferation and differentiation of markers under proinflammatory conditions was analyzed. Differentiation of the C2C12 line was completed at 4 days, corresponding with previous studies (Figure 6). It was found that Pax7 expression was highest in the first days of differentiation and was lost by day 4 (64.7±5% control vs 100% basal, p<0.05, n=5, Figure 6A) thereby increasing MHC (757.6±85.7% control vs 100% basal, p<0.001, n=5, Figure 6B). Maintenance of PAX7 expression was observed during the 4 days (121±8% IL-6, 114±11% INFγ, 98.1±10.5% LPS vs 100% basal at day 4, p<0.05, n=5, Figure 6A) and absence of MHC expression on day 4 (127.7±15.8% IL-6, 123.1±20.3% INFγ, 128.6±17% LPS vs 757.6±85% control at day 4, p<0.05, n=5, Figure 6A) under pro-inflammatory conditions. Dipyridamole treatment alone did not modulate myogenesis (p=ns) but did reverse PAX7 (64.5±5.8% IL-6+Dipyridamole vs 121±8% IL-6, 57.6±8.7% INFγ+Dipyridamole vs 114±11% INFγ, 60.7±11.4% LPS+Dipyridamole vs 98.1±10.5% LPS, p<0.05, n=5, Figure 6A) and MHC (614.6±92% IL-6+Dipyridamole vs 127.7±15.8% IL-6, 359±48% INFγ+Dipyridamole vs 123.1±20.3% INFγ, 460±69.6% LPS+Dipyridamole vs 128.6±17% LPS, p<0.05, n=5, Figure 6B) expression levels at day 4.

As it was previously elucidated that the therapeutic role of dipyridamole in muscle was due to increased expression of the A2B adenosine receptor and activation of the cAMP pathway, this pathway was tested. The inflammatory environment led to a loss of A2BR expression at 24h (75.8±2.2% IL-6, 69.3±1.9% INFγ, 70.8±5% LPS vs 112.9±4.7% control at 24h, p<0.05, n=5, Figure 6C) and 4 days after differentiation (67.6±3% IL-6, 66.03±20.3% INFγ, 56.5±5.5% LPS vs 172.7±12.6% control at 24h, p<0.05, n=5, Figure 6C). Dipyridamole stimulated A2BR expression at both 24h (116.1±4.2% IL-6+Dipyridamole vs 75.8±2.2% IL-6, 124.5±12.4% INFγ+Dipyridamole vs 69.3±1.9% INFγ, 13±8.4% LPS+Dipyridamole vs 70.8±5% LPS, p<0.05, n=5, Figure 6C) and 4 days (212±20.3% IL-6+Dipyridamole vs 67.6±3% IL-6, 173.6±15.4% INFγ+Dipyridamole vs 66.03±20.3% INFγ, 164±9.7% LPS+Dipyridamole vs 56.5±5.5% LPS, p<0.05, n=5, Figure 6C) of differentiation reversing the suppressive effect of pro-inflammatory cytokines. At day 4 of differentiation the use of pro-inflammatory cytokines reduced the expression of PKAα (107±5.8% IL-6, 101.7±3.7% INFγ, 95±5.3% LPS vs 213.9±21.6% control at 4 days, p<0.05, n=5, Figure 6D). Dipyridamole increased PKAα expression at both 24h (185.5±35.9% dipyridamole vs 126.5±20.2% control, p<0.05, n=5, Figure 6D) and 4 days after differentiation (245.3±34.3% dipyridamole vs 213.9±21.6% control, p<0.05, n=5, Figure 6D). Dipyridamole reversed PKAα levels at day 4 when combined with IL-6 (245.3±34.3% dipyridamole vs 107±5.8% IL-6, p<0.05, n=5, Figure 6D) and INFγ (245.3±34.3% dipyridamole vs 101.7±3.7% INFγ, p<0.05, n=5, Figure 6D). CREB activation is increased with muscle differentiation (357.7±45.15% control at day 4 vs 100% basal, p<0.05, n=5, Figure 6E). The use of pro-inflammatory cytokines inhibits CREB activation at day 4 (163.4±19.26% IL-6, 176.7±27.8% INFγ, 177±27.8% LPS vs 353.7±45.15% control at day 4, p<0.05, n=5, Figure 6E). On the other hand, dipyidamole increases CREB expression after 24h of differentiation (361.5±68.8% dipyridamole vs 100% basal, p<0.05, n=5, Figure 1E) and allows the reversal of CREB levels lost by pro-inflammatory cytokines at day 4 of differentiation (386.4±80.2% IL-6+Dipyridamole vs 163.4±19.26% IL-6, 304±37.3% INFγ+Dipyridamole vs 176.7±27.8% INFγ, 340.8±34.15% LPS+Dipyridamole vs 177±27.8% LPS, p<0.05, n=5, Figure 6E).

In this study the inventors found that AMPK was activated with muscle differentiation (222.7±23.9% control vs 100% basal, p<0.05, n=5, Figure 6F). At day 4 the use of proinflammatory cytokines inhibited AMPK activation (80.9±5.8% IL-6, 80.4±6.1% INFγ, 51.3±11.9% LPS vs 222.7±23.9% control at day 4, p<0.05, n=5, Figure 6F). Dipyridamole did not increase AMPK levels but reversed the inactivation observed with pro-inflammatory cytokines (215±33.3% IL-6+Dipyridamole vs 80.9±5.8% IL-6, 172.3±16.5% INFγ+Dipyridamole vs 80.4±6.1% INFγ, 175.8±26.9% LPS+Dipyridamole vs 51.3±11.9% LPS, p<0.05, n=5, Figure 6F).

### Example 8: Dipyridamole reduces inflammation and weight loss in experimental arthritis

In their quest for a model of inflammatory arthritis which could display associated sarcopenia the inventors employed serum transfer from K/BxN progeny in 12-wk-old C57BL/6J mice. Arthritic K/BxN mice were generated by crossing KRN mice with NOD/Lt mice. Serum was collected and pulled. Age-matched, male recipient, 12-wk-old C57BL/6J mice were purchased from Charles River Laboratories (Barcelona, España). C57BL/6J mice were injected with pooled serum (100 µ!, I.P.) from K/BxN mice on day 0 and 2. Dipyridamole was administered daily for up to 2 wk (25 and 50 mg/Kg).

Development of arthritis was assessed daily, and the severity of arthritis was assessed in each paw on a 4-point scale defined as follows: 0 = normal appearance, 1 = localized edema/erythema over one surface of the paw, 2 = edema/erythema involving more than one surface of the paw, and 3-4 = marked edema/erythema involving the whole paw. Scores of all 4 paws were added for a composite score. All motor activity tests were performed prior to serum inoculation and at the highest point of inflammation. Mice were euthanized on day 14. Gastronemius and cuadriceps were collected for protein and RNA extraction. Tibialis and soleus were included for freeze histology following the indications of H. Meng (1). Bones were prepared for micro-computed tomography (microCT) and histology. White adipose tissue (WAT) and brown adipose tissue (BAT) were collected for histology and protein extraction. Blood was drawn and serum was isolated for measurement of inflammatory cytokines and C-reactive protein (CPR).

As previously described for this model, paw thickness and inflammation had its highest peak at 2 wk after injection of K/BxN serum (Figure 7A). The use of dipyridamole prevented the appearance of thickness (0.5±0.01mm dipyiridamole vs 1.72±0.09mm arthritic, p<0.05, n=7) (Figure 7A) and inflammation (clinical score of 5±3 dipyiridamole vs 14±1 arthritic, p<0.005, n=7) (Figure 7B). On the other hand, arthritic mice showed a weight loss during the first week (8.8±3.8% weight loss vs sham, p<0.05, n=4-7) (Figure 7D) that was enhanced during the second week (14.2±4.1% weight loss vs sham, p<0.001, n=4-7) (Figure 7D) consistent with sarcopenia. Dipyridamole treatment prevented this weight loss during the 2 weeks of treatment (6.2±3.4% weight loss vs 14.2±4.1% for arthritic, p<0.01, n=7) been non-significant compared to sham mice (Figure 7D).

### Example 9: Dipyridamole treatment is able to revert the loss of muscle activity induced by experimental arthritis

Physical activity tests were performed to assess the motor development of the mice during the arthritic process as follows:

### 1. Ambulatory activity

Ambulation in 12-week-old male C57BL/6 mice was recorded following the protocol described by Gibbs and Crosbie-Watson (2). Mice were placed in individual recording chambers and were allowed to freely move for 6 min. Distance traveled by each animal was determined using object tracking in Kinovea open-source video analysis software (V 0.9.5). Data are represented as the difference between baseline and after 2 weeks of serum administration ± dipyridamole 25 and 50 mg/Kg/day.

During the ambulatory activity test, it was observed that arthritic mice reduced the distance travelled (830±37cm arthritic vs 1393±187cm sham, p<0.001, n=4-7) (Figure 8A). Treatment with dipyridamole helped mice to travel a similar distance as sham mice (1203±53cm dipyridamole vs 1393±187cm sham, p=ns, n=7) (Figure 8A).

### 2.Weights test

The protocol proposed by M. J. Deacon was used, adapting the weights to 17, 26, 35, 44, 53, 62 and 70g. Each mouse was given 3 opportunities to maintain weight. The weight assay was performed at day 0 and 12.

During the weights test sham mice were able to lift a similar weight among the two 2 weeks tested (44±4g week 1 vs 40±7g week 2 p=ns, n= 4) (Figure 8B). Induction of arthritis affected the strength of the mice, reducing weight bearing after two weeks (23±7g arthritic vs 41±5g sham, p<0.005, n= 4-7) (Figure 8B). Dipyridamole treatment prevented the loss of strength during the 2 weeks of arthritis induction (42±6g dipyiridamole vs 23±7g arthritic, p<0.05, n = 7) (Figure 8B).

### 3. Two limb hanging test

The test was performed at baseline and after 2 weeks of serum administration ± dipyridamole 25 and 50 mg/Kg/day maintaining the parameters established by Aartsma-Rus and van Putten (75).

The two-limb hanging test showed that arthritic mice had loss physical endurance (average score 3.6±3.3 arthritic vs 12±3 sham, p<0.005, n= 4-7) (Figure 8C). This loss was prevented by dipyridamole (average score 9.8±2 dipyridamole vs arthritic, p<0.05, n= 7) (Figure 8C).

### 4. Rotarod

The coordinated motor function of the four limbs was evaluated with the rotarod test (Rotarod, Harvard Apparatus, USA) with male C57BL/6 mice at the age of 12-week-old. Burgos et al. protocol was followed with modifications (76). Mice were first familiarized with the rotarod by letting them stay for 120s on the rod rotating at 4 and 6 rotations per minute (rpm). Motor activity was assessed by training the mice with an accelerated rotarod protocol of three trials, during which the rod accelerated from 4 rpm to 40 rpm within 5 min. All trials had a maximal duration of 300s and were interrupted when the mouse fell, or it stopped walking on the rotating rod.

Arthritic mice had an increased latency to fall (43.3±5.4seg arthritic vs 75.9±3.3seg sham, p<0.05, n=4-7) (Figure 8D), with Dipyridamole treatment been able to recover it (73.3±10.9seg dipyridamole vs 43.3±5.4seg arthritic, p<0.05, n=7) (Figure 8D).

### Example 10: Dual-energy x-ray absorptiometry (DXA)

Previously to arthritis induction and sacrifice day, all mice underwent DXA scan. For DXA analysis, a Lunar PIXlmus densitometer (software version 2.10) was calibrated using a hydroxyapatite phantom provided by the manufacturer (Lunar Corp, Madison, Wl). Subsequently, mice were anesthetized and whole-body scans were conducted. Data analysis consisted of whole body lean mass, fat mass, bone mineral density and bone mineral content. The results can be seen in Figure 9.

### Example 11: Dipyridamole restores muscle structure loss in experimental arthritis

Analysis of tibialis tissue structure by Hematoxylin and eosin (H&E) staining was performed. Frozen samples were sectioned at 10µm. For staining and subsequent processing by image segmentation we followed the indications established by Liu et al (53). Briefly, sections were brought from -80ºC to room temperature and immediately immersed in methanol for 30 min to improve fixation and stained with hematoxylin and eosin. Image analysis was performed with ImagePro 2.2 using the Muscle Morphometry plugin following Liu's protocol.

The presence of fibrosis is highlighted (black arrow, Figure 10A), in addition to a greater separation between fibers, that were reverted in the presence of Dipyridamole. Moreover, Dipyridamole treated muscles showed central nuclei in the fibers that may indicate an active muscle remodelling (white arrow, Figure 10A). When muscle fiber area was analysed, it was observed that this was smaller in arthritic mice when compared to sham (508±20µm² vs 1211±43µm² Sham, p<0.001, n=4-7) (Figure 10B). This loss of muscle fiber size was prevented by treatment with dipyridamole (1305±35µm² dipyridamole vs 508±20µm² arthritic, p<0.001, n=7) (Figure 10B).

Table 1 (below) shows the body composition of sham, arthritic and dipyridamole mice. Data collected for absolute weight are correlated with mouse weight to obtain relative weight (* indicates differences with the sham group and $ indicates differences with the arthritic group). WAT, white adipose tissue; BAT, brown adipose tissue. Values are presented as mean ± SEM.

| **Study groups** | **N** | | **Absolute weight (g)** | **Relative weight (% of body weight)** |
|---|---|---|---|---|
| **Sham** | 4 | Body weight | 23.22 ± 0.68 | / |
| | | Cuadriceps | 0.16 ± 0.01 | 0.73 ± 0.03 |
| | | Gastronemius | 0.142 ± 0.002 | 0.613 ± 0.017 |
| | | Soleus | 0.011 ± 0.001 | 0.05 ± 0.003 |
| | | Tibialis | 0.07 ± 0.005 | 0.3 ± 0.02 |
| | | WAT | 0.25 ± 0.023 | 1.074 ± 0.071 |
| | | BAT | 0.13 ± 0.01 | 0.556 ± 0.029 |
| **Arthritic** | 7 | Body weight | 23.02 ± 0.60 | / |
| | | Cuadriceps | 0.09 ± 0.01 | 0.42 ± 0.02^{∗∗∗} |
| | | Gastronemius | 0.118 ± 0.006 | 0.513 ± 0.02^{∗} |
| | | Soleus | 0.007 ± 0.001 | 0.03 ± 0.003^{∗} |
| | | Tibialis | 0.049 ± 0.005 | 0.21 ± 0.04^{∗} |
| | | WAT | 0.385 ± 0.019 | 1.67 ± 0.05^{∗∗} |
| | | BAT | 0.116 ± 0.006 | 0.501 ± 0.029^{∗∗} |
| **Dipyridamole** | 6 | Body weight | 22.12 ± 0.18 | / |
| | | Cuadriceps | 0.15 ± 0.01 | 0.69 ± 0.02 $$$ |
| | | Gastronemius | 0.123 ± 0.004 | 0.556 ± 0.011 $ |
| | | Soleus | 0.008 ± 0.001 | 0.04 ± 0.002 |
| | | Tibialis | 0.056 ± 0.004 | 0.263 ± 0.018$ |
| | | WAT | 0.198 ± 0.018 | 0.894 ± 0.076 $$$ |
| | | BAT | 0.157 ± 0.005 | 0.708 ± 0.026 ^{∗} / $$ |

### Example 12: Dipyridamole activates muscle regeneration via adenosine A2A/A2B receptors, AMPK activation and prevents muscle death in experimental arthritis

Protein expression analyses in gastronemius showed a non-significant increase in the muscle proliferation protein Pax7 in arthritic mice when compared to sham (151±13% arthritic vs 100±1% sham, p=ns, n=4-7), that was enhanced in the presence of dipyridamole (274±11% dipyridamole vs 151±13% arthritic, p<0.001, n=7) (Figure 11A). The muscle differentiation protein MHC decreased with the induction of arthritis (60±7% arthritic vs 100±1% sham, p<0.01, n=4-7), however the use of dipyridamole increased its expression (184±6% dipyridamole vs 100±1% sham, p<0.001, n=4-7) (Figure 11A).

On the other hand, cAMP-stimulating adenosine receptors A2A and A2B were significantly decreased in arthritic mice (71±3% for A2A and 35±9% for A2B vs 100±1% Sham, p<0.05 respectively, n=4-7), with Dipyridamole treatment enhancing the expression of both receptors (123±6% for A2A and 160±14% for A2B vs. 100±1% Sham, p=ns, n=4-7) (Figure 11B).

With arthritis induction an inactivation of AMPK was observed, with a decreased in phosphorylation (53±7% arthritic vs 100±1% sham, p<0.05, n=4-7), which was prevented with Dipyridamole (152±5% dipyridamole vs 100±1% sham, p=0.001, n=4-7) (Figure 12A).

As muscle in arthritic mice was observed to decrease in its differentiated state but not to increase proliferation, an analysis of muscle senescence markers p21 and p16 was performed. Both p21 and p16 protein expression were increased in arthritic mice (221±38% for p21 and 392±25% for p16 vs 100±1% sham, p<0.05 and p<0.001 respectively, n=4-7), been Dipyridamole able to decreased both expressions (64±15% for p21 vs 221±38% arthritic, p=0.01, and 139±32% for p16 vs 392±25% arthritic, p=0.001, n=7) (Figures12B&C).

### Example 13: Systemic inflammation induced in arthritis is prevented by Dipyridamole

Having proven that dipyridamole prevents sarcopenia associated with the arthritic process, the inventors wanted to know if this was associated with an improvement of the systemic inflammatory process associated to the induction or arthritis. For this purpose, they performed an array of protein expression of pro- and anti-inflammatory cytokines and chemokines in serumby using the Mouse Cytokine Array Q1 (RayBiotech, EEUU) which, simultaneously, detects 20 cytokines. The cytokines concentration was measured by fluorescence using GenePix following manufacture protocol for serum samples.

CRP concentration was measured using the colorimetric Mouse CRP ELISA kit following manufacture protocol for serum samples (Crystal Chem, USA). Absorbance was measured at 450 nm using a microplate reader Tecan Spark 20M.

No differences in CRP were found among groups (2861±893 ng/ml sham vs 4098±560ng/ml arthritic vs 3543±586 ng/ml dipyridamole, p=ns, n=4-7 although a tendency to decrease was observed in Dipyridamole treated mice (Figure13A). Increased values of pro-inflammatory cytokines INFγ (122.3±9.9 pg/ml arthritic vs 38.1±4.5 pg/ml sham, p<0.001, n=4-7) (Figure 13B), IL-1β (15.3±3.2 pg/ml arthritic vs 0.6±0.7 pg/ml sham, p<0.01, n=4-7) (Figure 13C), TNFα (44±5.4 pg/ml arthritic vs 21.53 pg/ml sham, p<0.05, n=4-7) (Figure 13D) and IL-6 (38.4±4.6 pg/ml arthritic vs 19.9±2.1 pg/ml sham, p<0.05, n=4-7) (Figure 13E) were observed in arthritic mice.

Dipyridamole treatment was able to decreased INFγ (59.6±7.6 pg/ml dipyridamole vs 122.3±9.9 pg/ml arthritic, p<0.001, n=6-7) (Figure 13B), IL-1β(2.3±0.8 pg/ml dipyiridamole vs 15.3±3.2 pg/ml arthritic, p<0.01, n=6-7) (Figure 13C), TNFα (24.9±3.8 pg/ml dipyiridamole vs 44±5.4 pg/ml arthritic, p<0.05, n=6-7) (Figure 13D) and IL-6 (23.4±2.1 pg/ml dipyiridamole vs 38.4±4.6 pg/ml arthritic, p<0.05, n=6-7) (Figure 13E). On the other hand, the analysis showed that proinflammatory cytokines and chemokines IL-2, IL-3, IL-12, IL-17 and RANTES (Figures 8F-J) showed no differences between groups (p=ns).

In the analysis of anti-inflammatory cytokines and chemokines, we observed that arthritis induced an increased in IL-10 when compared to sham (117.6±5.2 pg/ml arthritic vs 72.7±14.1 pg/ml sham, p<0.05, n=4-7) (Figure 14A), but not IL-9 (4.3±1.8 pg/ml arthritic vs 0.3±2 pg/ml sham, p=ns, n=4-7) (Figure 14B), dipyridamole been able to enhanced their expression (166.2±10 pg/ml for IL-10 vs 117.6±5.2 pg/ml arthritic, and 13.1±2.6 pg/ml for IL-9 vs 4.3±1.8 pg/ml, p=0.005 and p=0.05, respectively, n=7) (Figure 14A and 14B). No differences were observed for IL-4, IL-5 and IL-13 (Figures 14C-E). On the other hand, angiogenesis, vascular endothelial growth factor (VEGF), was reduced in arthritic mice (90±13.1 pg/ml vs 139.2±12 pg/ml Sham, p, n=4-7) and enhanced in the presence of dipyridamole (221.1±40.3 pg/ml vs 90±13.1 pg/ml arthritic, p=0.05, Figure 14F).

### REFERENCES

1.- Dhillon, R.J.S.; Hasni, S. Pathogenesis and Management of Sarcopenia. Clin. Geriatr. Med. 2017, 33, 17-26.
2.- Janssen I, Shepard DS, Katzmarzyk PT, Roubenoff R. The Healthcare Costs of Sarcopenia in the United States: ECONOMIC COST OF SARCOPENIA. Journal of the American Geriatrics Society. 2004;52(1):80-5.
3.- Rolland Y, Czerwinski S, van Kan GA, Morley JE, Cesari M, Onder G, et al. Sarcopenia: Its assessment, etiology, pathogenesis, consequences and future perspectives. J Nutr Health Aging. 2008;12(7):433-50.
4.- Masanés Torán F, Navarro López M, Sacanella Meseguer E, López Soto A. ¿Qué es la sarcopenia? Seminaries de la Fundación Española de Reumatologia. 2010;11(1):14-23.
5.- Cruz-Jentoft AJ, Baeyens JP, Bauer JM, Boirie Y, Cederholm T, Landi F, et al. Sarcopenia: European consensus on definition and diagnosis: Report of the European Working Group on Sarcopenia in Older People. Age and Ageing. 2010;39(4):412-23.
6.- Fielding RA, Vellas B, Evans WJ, Bhasin S, Morley JE, Newman AB, et al. Sarcopenia: An Undiagnosed Condition in Older Adults. Current Consensus Definition: Prevalence, Etiology, and Consequences. International Working Group on Sarcopenia. Journal of the American Medical Directors Association. 2011;12(4):249-56.
7.- Muscaritoli M, Anker SD, Argilés J, Aversa Z, Bauer JM, Biolo G, et al. Consensus definition of sarcopenia, cachexia and pre-cachexia: Joint document elaborated by Special Interest Groups (SIG) "cachexia-anorexia in chronic wasting diseases" and "nutrition in geriatrics". Clinical Nutrition. 2010;29(2):154-9.
8.- Cannataro R, Carbone L, Petro JL, Cione E, Vargas S, Angulo H, et al. Sarcopenia: Etiology, Nutritional Approaches, and miRNAs. IJMS 2021;22(18):9724
9.- Little RD, Prieto-Potin I, Pérez-Baos S, Villalvilla A, Gratal P, Cicuttini F, et al. Compensatory anabolic signaling in the sarcopenia of experimental chronic arthritis. Sci Rep. 2017;7(1):6311.
10.- Sharma B, Dabur R. Role of Pro-inflammatory Cytokines in Regulation of Skeletal Muscle Metabolism: A Systematic Review. Current Med Chem. 2020;27(13):2161-88.
11.- Aleman H, Esparza J, Ramirez FA, Astiazaran H, Payette H. Longitudinal evidence on the association between interleukin-6 and C-reactive protein with the loss of total appendicular skeletal muscle in freeliving older men and women. Age and Ageing. 2011;40(4):469-75.
12.- Hofmann SR, Rösen-Wolff A, Tsokos GC, Hedrich CM. Biological properties and regulation of IL-10 related cytokines and their contribution to autoimmune disease and tissue injury. Clinical Immunology. 2012;143(2):116-27.
13.- Heredia JE, Mukundan L, Chen FM, Mueller AA, Deo RC, Locksley RM, et al. Type 2 Innate Signals Stimulate Fibro/Adipogenic Progenitors to Facilitate Muscle Regeneration. Cell. 2013;153(2):376-88.
14.- Chang YH, Tsai JN, Chen TL, Ho KT, Cheng HY, Hsiao CW, et al. Interleukin-4 Promotes Myogenesis and Boosts Myocyte Insulin Efficacy. Mediators of Inflammation. 2019;2019:1-14.
15.- Pérez-Baos S, Prieto-Potin I, Román-Blas JA, Sánchez-Pernaute O, Largo R, Herrero-Beaumont G. Mediators and Patterns of Muscle Loss in Chronic Systemic Inflammation. Front Physiol. 2018;9:409.
16.- Singh JA, Saag KG, Bridges SL, Akl EA, Bannuru RR, Sullivan MC, et al. American College of Rheumatology Guideline for the Treatment of Rheumatoid Arthritis: ACR RA TREATMENT RECOMMENDATIONS. Arthritis & Rheumatology 2016;68(1):1-26.
17.- Firestein GS. Evolving concepts of rheumatoid arthritis. Nature. 2003;423(6937):356-61.
18.- Bresnihan B. Pathogenesis of joint damage in rheumatoid arthritis. J Rheumatol. 1999;26:717-9.
19.- Michaud K, Wolfe F. Comorbidities in rheumatoid arthritis. Best Practice & Research Clinical Rheumatology. 2007;21(5):885-906.
20.- Ångström L, Hörnberg K, Sundström B, Södergren A. Rheumatoid cachexia in early rheumatoid arthritis: prevalence and associated variables. Scandinavian Journal of Rheumatology. 2021;1-7.
21.- Roubenoff R, Roubenoff RA, Cannon JG, Kehayias JJ, Zhuang H, Dawson-Hughes B, et al. Rheumatoid cachexia: cytokine-driven hypermetabolism accompanying reduced body cell mass in chronic inflammation. J Clin Invest. 1994;93(6):2379-86.
22.- Morley JE, Thomas DR, Wilson MMG. Cachexia: pathophysiology and clinical relevance. The American Journal of Clinical Nutrition 2006;83(4):735-43.
23.- Santo RCE, Fernandes KZ, Lora PS, Filippin LI, Xavier RM. Prevalence of rheumatoid cachexia in rheumatoid arthritis: a systematic review and meta-analysis: Systematic Review of RA cachexia prevalence. Journal of Cachexia, Sarcopenia and Muscle. 2018;9(5):816-25.
24.- Webster JM, Kempen UAP, Hardy RS, Langen RCJ. Inflammation and Skeletal Muscle Wasting During Cachexia. Front Physiol. 2020;11:597675.
25.- Schiaffino S, Dyar KA, Ciciliot S, Blaauw B, Sandri M. Mechanisms regulating skeletal muscle growth and atrophy. FEBS Journal. 2013: 280(17):4294-314
26.- Lemmey AB, Wilkinson TJ, Clayton RJ, Sheikh F, Whale J, Jones HSJ, et al. Tight control of disease activity fails to improve body composition or physical function in rheumatoid arthritis patients. Rheumatology (Oxford). 2016 Oct;55(10):1736-45.
27.- Burnstock G. The past, present and future of purine nucleotides as signalling molecules. Neuropharmacology. 1997;36(9):1127-39.
28.- Fredholm BB, IJzerman AP, Jacobson KA, Linden J, Müller CE. International Union of Basic and Clinical Pharmacology. LXXXI. Nomenclature and Classification of Adenosine Receptors-An Update. Pharmacol Rev. marzo de 2011;63(1):1-34.
29.- Verzijl D, IJzerman AP. Functional selectivity of adenosine receptor ligands. Purinergic Signalling. 2011;7(2):171-92.
30.- Burnstock G. Purine and pyrimidine receptors. Cell Mol Life Sci. 2007;64(12):1471-83.
31.- Weisman GA, Wang M, Kong Q, Chorna NE, Neary JT, Sun GY, et al. Molecular Determinants of P2Y<SUB>2</SUB> Nucleotide Receptor Function: Implications for Proliferative and Inflammatory Pathways in Astrocytes. Mol Neurobiol 2005;31(1-3):169-84.
32.- Bornø A, Ploug T, Bune LT, Rosenmeier JB, Thaning P. Purinergic receptors expressed in human skeletal muscle fibres. Purinergic Signalling. 2012;8(2):255-64.
33.- Lynge, Hellsten. Distribution of adenosine A 1, A 2A and A 2B receptors in human skeletal muscle: Adenosine receptors in human skeletal muscle. Acta Physiologica Scandinavica. 2000;169(4):283-90.
34.- Hellsten Y, Maclean D, Rådegran G, Saltin B, Bangsbo J. Adenosine Concentrations in the Interstitium of Resting and Contracting Human Skeletal Muscle. Circulation. 1998;98(1):6-8.
35.- Zhao Y, Fabris S, MacLean DA. The effects of adenine nucleotide perfusion on interstitial adenosine production in rat skeletal muscle. Can J Physiol Pharmacol. 2018;96(8):823-9.
36.- Vergauwen L, Hespel P, Richter EA. Adenosine receptors mediate synergistic stimulation of glucose uptake and transport by insulin and by contractions in rat skeletal muscle. J Clin Invest. 1994;93(3):974-81.
37.- Gnad T, Navarro G, Lahesmaa M, Reverte-Salisa L, Copperi F, Cordomi A, et al. Adenosine/A2B Receptor Signaling Ameliorates the Effects of Aging and Counteracts Obesity. Cell Metabolism. 2020;32(1):56-70.e7.
38.- Siow NL, Choi RCY, Cheng AWM, Jiang JXS, Wan DCC, Zhu SQ, et al. A Cyclic AMP-dependent Pathway Regulates the Expression of Acetylcholinesterase during Myogenic Differentiation of C2C12 Cells. Journal of Biological Chemistry 2002;277(39):36129-36.
39.- Suarez-Berumen K, Collins-Hooper H, Gromova A, Meech R, Sacco A, Dash PR, et al. Pannexin 1 Regulates Skeletal Muscle Regeneration by Promoting Bleb-Based Myoblast Migration and Fusion Through a Novel Lipid Based Signaling Mechanism. Front Cell Dev Biol 2021;9:736813.
40.- Ishack S, Mediero A, Wilder T, Ricci JL, Cronstein BN. Bone regeneration in critical bone defects using three-dimensionally printed β-tricalcium phosphate/hydroxyapatite scaffolds is enhanced by coating scaffolds with either dipyridamole or BMP-2: AGENTS THAT STIMULATE A2A RECEPTORS FURTHER ENHANCE HA/B-TCP SCAFFOLDS BONE REGENERATION. J Biomed Mater Res. 2017;105(2):366-75.
41.- Mediero A, Wilder T, Perez-Aso M, Cronstein BN. Direct or indirect stimulation of adenosine A 2A receptors enhances bone regeneration as well as bone morphogenetic protein-2. FASEB j. 2015;29(4):1577-90.
42.- Mediero A, Wilder T, Reddy VSR, Cheng Q, Tovar N, Coelho PG, et al. Ticagrelor regulates osteoblast and osteoclast function and promotes bone formation in vivo via an adenosine-dependent mechanism. FASEB j. 2016;30(11):3887-900.
43.- Feig JL, Mediero A, Corciulo C, Liu H, Zhang J, Perez-Aso M, et al. The antiviral drug tenofovir, an inhibitor of Pannexin-1-mediated ATP release, prevents liver and skin fibrosis by downregulating adenosine levels in the liver and skin. PLoS One 2017;12(11):e0188135.
44.- Conesa-Buendía FM, Llamas-Granda P, Larranaga-Vera A, Wilder T, Largo R, Herrero-Beaumont G, et al. Tenofovir Causes Bone Loss via Decreased Bone Formation and Increased Bone Resorption, Which Can Be Counteracted by Dipyridamole in Mice. J Bone Miner Res. 2019;34(5):923-38.
45.- Firas M Kara, Violeta Chitu, Jennifer Sloane, Matthew Axelrod, Bertil B Fredholm, E Richard Stanley, Bruce N Cronstein. Adenosine A1 receptors (A1Rs) play a critical role in osteoclast formation and function. The FASEB Journal 2010; 24(7): 2325-2333.
46.- Firas M Kara, Stephen B Doty, Adele Boskey, Steven Goldring, Mone Zaidi, Bertil B Fredholm, Bruce N Cronstein. Adenosine A(1) receptors regulate bone resorption in mice: adenosine A(1) receptor blockade or deletion increases bone density and prevents ovariectomy-induced bone loss in adenosine A(1) receptor-knockout mice. Arthritis and Rheumatism 2010; 62(2):534-541.
47.- Joseph M Russell, Graham S Stephenson, Clare E Yellowley, Hilary P Benton. Adenosine inhibition of lipopolysaccharide-induced interleukin-6 secretion by the osteoblastic cell line MG-63. Calcif Tissue Int 2007; 81(4) :316-326.
48.- Bronwen A J Evans, Carole Elford, Annette Pexa, Karen Francis, Alis C Hughes, Andreas Deussen, Jack Ham. Human osteoblast precursors produce extracellular adenosine, which modulates their secretion of IL-6 and osteoprotegerin. J Bone Miner Res 2006; 21(2): 228-236.
49.- M Adelina Costa, A Barbosa, E Neto, A Sá-e-Sousa, R Freitas, J M Neves, T Magalhães-Cardoso, F Ferreirinha, P Correia-de-Sá. On the role of subtype selective adenosine receptor agonists during proliferation and osteogenic differentiation of human primary bone marrow stromal cells.Journal of Cellular Physiology 2011; 226(5): 1353-1366.
50.- Schuster-Gossler, K.; Cordes, R.; Gossler, A. Premature Myogenic Differentiation and Depletion of Progenitor Cells Cause Severe Muscle Hypotrophy in Delta1 Mutants. Proc. Natl. Acad. Sci. 2007, 104, 537-542.
51.- von Maltzahn, J; Jones, A.E.; Parks, R.J.; Rudnicki, M.A. Pax7 Is Critical for the Normal Function of Satellite Cells in Adult Skeletal Muscle. Proc. Natl. Acad. Sci. 2013, 110, 16474-16479.
52.- Liu, H.-W.; Chang, Y.-C.; Chan, Y.-C.; Hu, S.-H.; Liu, M.-Y.; Chang, S.-J. Dysregulations of Mitochondrial Quality Control and Autophagic Flux at an Early Age Lead to Progression of Sarcopenia in SAMP8 Mice. Biogerontology 2020, 21, 367-380.
53.- Ryten, M.; Dunn, P.M.; Neary, J.T.; Burnstock, G. ATP Regulates the Differentiation of Mammalian Skeletal Muscle by Activation of a P2X5 Receptor on Satellite Cells. J. Cell Biol. 2002, 158, 345-355.
54.- Orriss, I.R.; Knight, G.E.; Ranasinghe, S.; Burnstock, G.; Arnett, T.R. Osteoblast Responses to Nucleotides Increase during Differentiation. Bone 2006, 39, 300-309.
55.- Martinello, T.; Baldoin, M.C.; Morbiato, L.; Paganin, M.; Tarricone, E.; Schiavo, G.; Bianchini, E.; Sandonà, D.; Betto, R. Extracellular ATP Signaling during Differentiation of C2C12 Skeletal Muscle Cells: Role in Proliferation. Mol. Cell. Biochem. 2011, 351, 183-196.
56.- Miller, S.G.; Hafen, P.S.; Brault, J.J. Increased Adenine Nucleotide Degradation in Skeletal Muscle Atrophy. Int. J. Mol. Sci. 2019, 21, 88.
57.- Lynge; Hellsten Distribution of Adenosine A 1 , A 2A and A 2B Receptors in Human Skeletal Muscle: Adenosine Receptors in Human Skeletal Muscle. Acta Physiol. Scand. 2000, 169, 283-290.
58.- Gnad, T.; Navarro, G.; Lahesmaa, M.; Reverte-Salisa, L.; Copperi, F.; Cordomi, A.; Naumann, J.; Hochhäuser, A.; Haufs-Brusberg, S.; Wenzel, D.; et al. Adenosine/A2B Receptor Signaling Ameliorates the Effects of Aging and Counteracts Obesity. Cell Metab. 2020, 32, 56-70.e7.
59.- Iminen, A.; Kaarniranta, K. AMP-Activated Protein Kinase (AMPK) Controls the Aging Process via an Integrated Signaling Network. Ageing Res. Rev. 2012, 11, 230-241.
60.- Herzig, S.; Shaw, R.J. AMPK: Guardian of Metabolism and Mitochondrial Homeostasis. Nat Rev Mol Cell Biol 2018, 19, 121-135.
61.- Singh, N.; Shreshtha, A.K.; Thakur, M.S.; Patra, S. Xanthine Scaffold: Scope and Potential in Drug Development. Heliyon 2018, 4, e00829.
62.- Chen, A.E.; Ginty, D.D.; Fan, C.-M. Protein Kinase A Signalling via CREB Controls Myogenesis Induced by Wnt Proteins. Nature 2005, 433, 317-322.
63.- Bordeaux, R.; Stewart, R. CAMP Signaling in Skeletal Muscle Adaptation: Hypertrophy, Metabolism, and Regeneration. Am. J. Physiol.-Endocrinol. Metab. 2012, 303, E1-E17.
64.- Stewart, R.; Flechner, L.; Montminy, M.; Berdeaux, R. CREB Is Activated by Muscle Injury and Promotes Muscle Regeneration. PLoS ONE 2011, 6, e24714.
65.- Wang, Z.; Zhang, L.; Liang, Y.; Zhang, C.; Xu, Z.; Zhang, L.; Fuji, R.; Mu, W.; Li, L.; Jiang, J.; et al. Cyclic AMP Mimics the Anti-Ageing Effects of Calorie Restriction by Up-Regulating Sirtuin. Sci. Rep. 2015, 5, 12012.
66.- Hinkle, R.T.; Dolan, E.; Cody, D.B.; Bauer, M.B.; Isfort, R.J. Phosphodiesterase 4 Inhibition Reduces Skeletal Muscle Atrophy. Muscle Nerve 2005, 32, 775-781.
67.- Fredholm BB. Adenosine-a physiological or pathophysiological agent? J Mol Med. 2014;92(3):201-6.
68.- Haskó G, Linden J, Cronstein B, Pacher P. Adenosine receptors: therapeutic aspects for inflammatory and immune diseases. Nat Rev Drug Discov. 2008;7(9):759-70.
69.- Chan ESL, Fernandez P, Cronstein BN. Adenosine in inflammatory joint diseases. Purinergic Signalling. 2007;3(1-2):145-52.
70.- Chargé SBP, Rudnicki MA. Cellular and Molecular Regulation of Muscle Regeneration. Physiological Reviews. 2004;84(1):209-38.
71.- Moriscot A, Miyabara EH, Langeani B, Belli A, Egginton S, Bowen TS. Firearms-related skeletal muscle trauma: pathophysiology and novel approaches for regeneration. npj Regen Med. 2021;6(1):17.
72.- Azhar M, Wardhani BWK, Renesteen E. The regenerative potential of Pax3/Pax7 on skeletal muscle injury. J Genet Eng Biotechnol. 2022;20(1):143.
73.- Gnad T, Navarro G, Lahesmaa M, Reverte-Salisa L, Copperi F, Cordomi A, et al. Adenosine/A2B Receptor Signaling Ameliorates the Effects of Aging and Counteracts Obesity. Cell Metabolism. 2020;32(1):56-70.e7.
74.- Mediero A1,Tuere Wilder, Reddy VSR, Cheng Q, Tovar N, Coelho PG3, Witek L, Whatling C, Cronstein BN. Ticagrelor regulates osteoblast and osteoclast function and promotes bone formation in vivo via an adenosine-dependent mechanism. FASEB J 2016;30(11):3887-3900.
75.- Aartsma-Rus A, van Putten M. Assessing Functional Performance in the Mdx Mouse Model. JoVE. 2014;(85):51303.
76.- Burgos DF, Machio-Castello M, Iglesias-Cabeza N, Giráldez BG, González-Fernández J, Sánchez-Martin G, et al. Early Treatment with Metformin Improves Neurological Outcomes in Lafora Disease. Neurotherapeutics 2022. Online ahead of print.

## Claims

1. Dipyridamole, its salt or derivative, for use in a method of treatment, amelioration or prevention of muscular myogenesis disorders or inflammatory arthritis, comprising administering to a subject in need thereof a therapeutically effective amount of dipyridamole, its salt or derivative.

2. Dipyridamole, for the use of claim 1, wherein the muscular myogenesis disorder is induced by the ageing of the said subject.

3. Dipyridamole, for the use of claim 1, wherein the muscular myogenesis disorder is induced by inflammatory disease(s) present in the subject, preferably chronic inflammatory disease(s).

4. Dipyridamole, for the use of any one of claims 1 to 3, wherein the muscular myogenesis disorder is sarcopenia, preferably primary or secondary sarcopenia.

5. Dipyridamole for the use of claim 1, wherein the inflammatory arthritis is selected from rheumatoid arthritis (RA) or a type of spondyloarthropathy, such as for example psoriatic arthritis and Systemic Lupus Erythematosus (SLE).

6. Dipyridamole, for the use of claim 1, wherein the inflammatory arthritis is rheumatoid arthritis (RA) or rheumatoid cachexia (RC).

7. Dipyridamole, for the use of any one of the preceding claims, wherein dipyridamole is administered to the subject in need thereof at between 25 to 100 mg/kg/day, preferably at between 50 to 75 mg/kg/day.

8. Dipyridamole, for the use of any one of the preceding claims, wherein the administration route is oral, intravenous, topical, intramuscular, preferably the administration route is oral.

9. Dipyridamole, for the use of any one of the preceding claims, wherein dipyridamole is provided in a pharmaceutical composition further comprising pharmaceutically acceptable excipients.
